# EUROPEAN PATENT APPLICATION

(11) **EP 2 977 379 A1**
(43) Date of publication of application: **27.01.2016**
(21) Application number: 14770263.3
(22) Date of filing: 19.02.2014
(51) Int. Cl.: C07F 9/44, C07F 9/6558, A61K 31/664, A61K 31/665, A61P 31/12, A61P 31/16

(54) **ALKYL 2-{[(2R,3S,5R)-5-(4-AMINO-2-OXO-2 -PYRIMIDIN-1-YL)-3-HYDROXY- TETRAHYDRO-FURAN-2-YL-METHOXY]-PHENOXY-PHOSPHORYL-AMINO}-PROPIONATES, NUCLEOSIDE INHIBITORS OF HCV NS5B RNA-POLYMERASE, AND METHODS FOR PRODUCING AND USE THEREOF**

(30) Priority: 22.03.2013 RU 2013112744
(71) Applicant: Ivachtchenko, Alexandre Vasilievich, Moskovskaya obl. 141700 (RU); Asavi, LLC, Hallandale Beach, Florida 33009 (US)
(72) Inventor: IVACHTCHENKO, Alexandre Vasilievich, Dolgoprydnyj (RU)
(74) Representative: Patentanwälte Zellentin & Partner
(86) International application number: PCT/RU2014/000104
(87) International publication number: WO 2014/148949

(57) **Abstract**

Compounds of the general formula **1,** stereoisomers, pharmaceutically acceptable salts, and/or hydrates, solvates or crystalline forms thereof 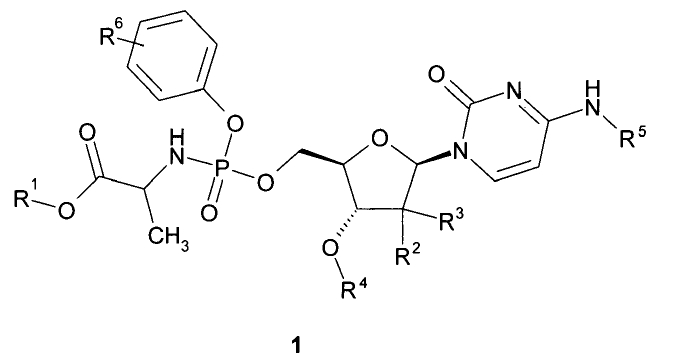 wherein
R¹ represents C₁-C₄ alkyl;
R² and R³ represents fluoro, or
R² represents fluoro, and R³ represents methyl;
R⁴ and R⁵ represents hydrogen, or
R⁴ represents C₁-C₆ acyl, and R⁵ represents hydrogen, or
R⁴ represents hydrogen, and R⁵ represents C₁-C₆ acyl, or
R⁴ represents optionally substituted α-aminoacyl, and R⁵ represents hydrogen, or
R⁴ represents hydrogen, and R⁵ represents optionally substituted α-aminoacyl;
R⁶ represents hydrogen, methyl, methoxy or halogen.

## Description

### Field of invention

The present invention relates to novel substituted alkyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionates, nucleoside inhibitors of HCV RNA-polymerase NS5B, their use as inhibitor of HCV RNA-polymerase NS5B and treatment of viral diseases. These compounds are inhibitors of RNA-dependent RNA viral replication and useful as nucleoside HCV NS5B polymerase inhibitors - inhibitors of hepatitis C virus (HCV) replication for treatment hepatitis C in mammalian body. Hepatitis C virus, as well as other important human pathogens, such as yellow fever virus, West Nile virus, Dengue virus and hepatitis GBV-C virus falls into the category of Flaviviridae (genus Flaviviridae).

### Background of the invention

The following nucleoside inhibitors of HCV NS5B polymerase may serve as examples of the drug candidates: PSI-7977 of Pharmaceut firm, USA (patents US 07964580 B2 and US 8334270 B2) and NM283 (Valopicitabine) of Idenix firm (USA) and others. [M. J. Sofia, D. Bao, W. Chang, J. Du, D. Nagarathnam, S. Rachakonda, P. G. Reddy, B. S. Ross, P. Wang, H.-R. Zhang, S. Bansal, C. Espiritu, M. Keilman, A. M. Lam, H. M. M. Steuer, Congrong Niu, M. J. Otto, P. A. Furman. Discovery of a β-D-20-Deoxy-20-r-fluoro-20-β-C-methyluridine Nucleotide Prodrug (PSI-7977) for the Treatment of Hepatitis C Virus. J. Med. Chem. 2010, 53, 7202-7218].

The most worked out drug candidate is nucleoside HCV NS5B polymerase inhibitor (*iso*-propyl (S)-2-{(S)-[(2R,3R,4R,5R)-5-(2,4-dioxo-3,4-dihydro-2H-pyrimidin-1-yl)-4-fluoro-3-hydroxy-4-methyl-tetrahydro-furan-2-ylmethoxy]-phenoxy-phosphorylamino}-propanoate (PSI-7977). Gilead Sciences is successful in carrying out clinical trials in treatment of HCV genotypes of 1, 2, and 3 using PSI-7977 - nucleotide analogue of polymerase inhibitor.

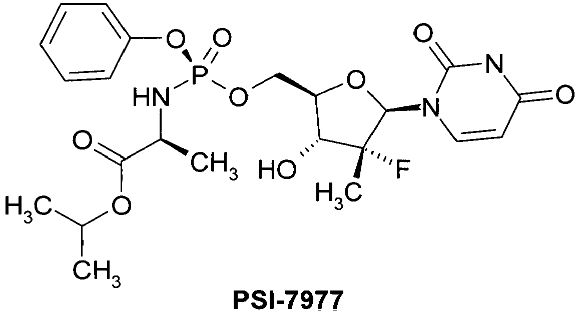

Experimental preparation GS-7977 of Gilead Sciences Inc company together with Daclatasvir (Bristol-Myers Squibb Co) gave positive result in 100% of patients suffering viral hepatitis C, who had previously not been treated with it, according to intermediate data of clinical study, represented at the European conference on hepatic diseases. It is important that the result has been achieved without employment of interferon, which has many side effects that sometimes bring patients to interrupt or postpone treatment, and of Ribavirin, which is usually a part of most classical therapeutic sequence.

Now there are a restricted number of approved therapeutic approaches, which would have been recognized suitable for treatment of HCV infection. New and already existing therapeutic approaches for treatment of HCV and inhibition of HCV NS5B polymerase are described in the following articles: R.G.Gish, Sem. Liver. Dis., 1999 19:5; Di Besceglie, A.M. And Bacon, B. R., Scientific American, October: 1999 80-85; G. Lake-Bakaarm Current and Future Therapy for Chronic Hepatitis C Virus Liver Disease, Curr. Drug Targ Infect Dis. 2003 3(3):247-253; P. Hoffmann et al. Recent patents on experimental therapy for hepatitis C vims infection (1999-2002), Exp.Opin. Ther. Patents 2003 13(11):1707-1723; F. F. Poordad et al. Developments in Hepatitis C therapy drug during 2000-2002, Exp.Opin. Emerging Drugs 2003 8(1):9-25; M.P. Walker et al. Promising Candidates for the treatment of chronic hepatitis C, Exp.Opin. Investig. Drugs 2003 12(8): 1269-1280; S. - L. Tan et al. Hepatitis C Therapeutics: Current Status and Emerging Strategies, Natire Rev. Drug Discov. 2002 1:867-881; R. De Francesco et al. Approaching a new era for hepatitis C virus therapy: inhibitors of the NS3-4A serine protease and the NS5B RNA-dependent RNA polymerase. Antiviral Res. 2003 58:1-16; Q.M. Wang et al. Hepatitis C virus encoded proteins: targets for antiviral therapy. Drugs of the Future 2000 25(9):933-8-944; J. A. Wu and Z. Hong, Targeting NS5B-Dependent RNA Polymerase for Anti-HCV Chemotherapy Cur. Dmg Targ.-Inf. Dis. 2003 3:207-219.

Nucleoside inhibitors of NS5B polymerase could act either as substrate of unnatural origin, that as a result bring about chain termination, or as a competitive inhibitor, the action of which is in competition with binding of nucleotide with polymerase. In order to operate as a chain terminator nucleoside analogue should have been absorbed by cell and *in vivo* converted in triphosphate to be able to compete for the site of nucleotide binding of polymerase. This conversation in triphosphate usually takes place with participation of cell kinases that accounts for additional structural requirements to potential nucleoside inhibitor of polymerase. Unfortunately, this restricts direct valuation of nucleosides as inhibitors of HCV replication in investigations carried out on cells subjecting *in situ* to phosphorylation. 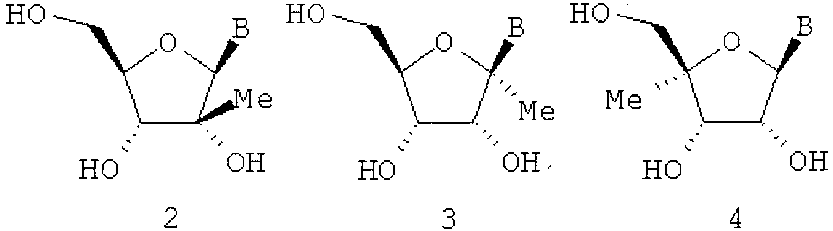 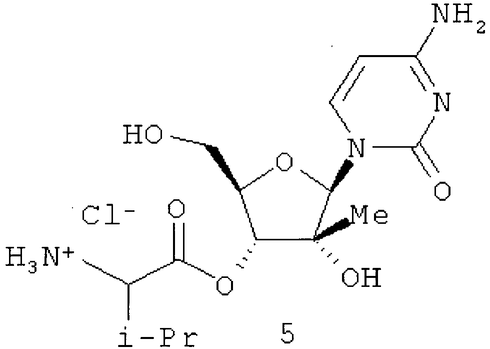 B = adenine, thymidine, uracil, cytidine, guanine and hypoxanthine.

In WO 0190121, published on the 29th of November 2001, J.-P.Sommadossi and P.Lacolla described and gave examples of anti-HCV polymerase activity of 1' - alkyl- and 2' -alkyl nucleosides of formulas 2 and 3. In WO 01/92282, published on the 6th of December 2001, J.-P.Sommadossi and P.Lacolla disclosed and described examples of treatment of Flaviviruses and Pestiviruses by means of 1' - alkyl- and 2' - alkyl nucleosides of formulas 2 and 3. In WO 03/026675, published on the 3 of April 2003, G. Gosselin described application of 4' - alkyl nucleosides of formula 4 for treatment of Flaviviruses and Pestiviruses.

In WO 2004003000, published on the 8th of January 2004, J.-P.Sommadossi et al. described 2' - and 3' -prodrugs on the base of 1' -, 2' -, 3' - and 4' -substituted β-D and β-L-nucleosides. In WO 2004/002422, published on the 8th of January 2004, is described 2' -C-methyl-3' -O-valine ester of ribofuranosylcytidine for treatment infection of Flaviviridae. Idenix reported on clinical trials of related compound NM283, that, is expected, to be ester of valine 5 and analogue of 2 cytidine (B = cytosine). In WO 2004/002999, published on the 8th of January 2004, J.-P.Sommadossi et al. described a series of 2' or 3' prodrugs from 1', 2', 3' or 4' - branched nucleosides for treatment of flavivirus infections, including HCV infections.

In WO 2004/046331, published on the 3d of June 2004, J.-P.Sommadossi et al. described 2' -branched nucleosides and Flaviviridae mutation. In WO 03/026589, published on the 3d of April 2003, G.Gosselin et al. described methods for treatment of viral hepatitis C by means of 4' -modified nucleosides. In WO 2005009418, published on the 3d of February 2005, R.Storer et al. described purine nucleoside analogues for treatment diseases caused by Flaviviridae as well.

Other patent applications disclose usage of certain nucleoside analogues for treatment of viral hepatitis C. In WO 01/32153, published on the 10th of May 2001, R.Storer described nucleoside derivatives for treatment of viral diseases. In WO 01/60315, published on the 23d of August 2001, H.Ismaili et al. described methods for prophylaxis and treatment of infections caused by Flaviviruses with the help of nucleoside derivatives. In WO 02/18404, published on the 7th of Mach 2002, R.Devos et al. described 4' -substituted nucleosides for treatment of viral HCV. In WO 01/79246, published on the 25th of October 2001, K.A.Watanabe described derivatives of 2' or 3' - hydroxymethyl-nucleosides for treatment of viral diseases. In WO 02/32920, published on the 25th of April 2002, and in WO 02/48165, published on the 20th of June 2002, L.Stuyver et al. described nucleoside derivatives for treatment of viral diseases.

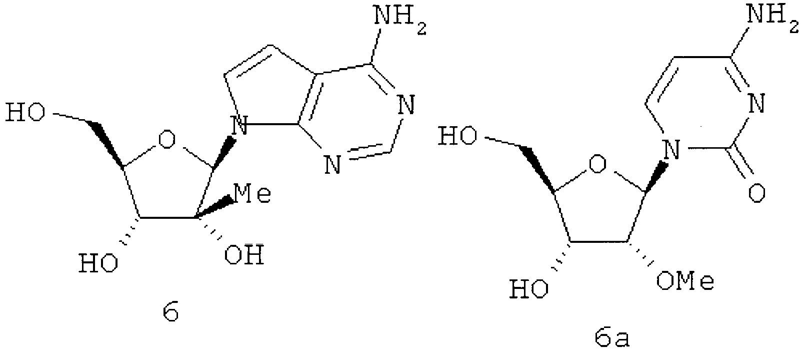

In WO 03/105770, published on the 24th of December 2003, B. Bhar et al. described a series of carbocyclic nucleoside derivatives, which inhibit RNA-dependent RNA-virus polymerase. Nucleosides disclosed in this publication are mainly 2' - methyl-2' -hydroxysubstituted nucleosides. In WO 2002/057425, published on the 25th of July 2002, S.S.Carroll et al. described nucleoside derivatives, which inhibit RNA-dependent viral polymerase, and methods for treatment of HCV diseases.

In WO 02/057287, published on the 25th of July 2002 r., S.S.Carroll et al. described related 2α -methyl and 2β -methylribose derivatives, in which the bases are not optionally substituted with 7H-pyrrolo[2,3-(1]pyrimidine radical 6. In the same application is given an example of 3β-methyl-nucleoside. S.S.Carroll et al. (J.Biol.Chem. 2003 278(14):11979-11984), described HCV polymerases inhibited by 2'-O-methylcytidines (6a). In WO 2004/009020, published on the 29th of January 2004, D.B.Olsen et al. described a series of thionucleoside derivatives as inhibitors of virus RNA-dependent RNA- polymerase.

PCT publication Nº WO 99/43691, in favour of Emory University, entitled "2'-Fluoronucleosides", describes application of certain 2'- fluoronucleosides for treating HCV. US patent Nº 6348587, granted to Emory University and entitled "2' - fluoronucleosides" discloses a number of 2'- fluoronucleoside families useful for treating hepatitis B, HCV, HIV and abnormal cell prolifiration. Both configurations of 2' -fluorosubstituent have been described.

Eldrup et al. (Oral Session V, Hepatitis C Virus, Flaviviridae; 16th International Conference on Antiviral Research (Apr. 27, 2003, Savannah, Ga.)) characterized the relation between the structure and activity for 2' - modified nucleosides in terms of HCV inhibition.

Bhat et al. (Oral Session V, Hepatitis C Virus, Flaviviridae; 16th International Conference on Antiviral Research (Apr. 27, 2003, Savannah, Ga.) p A75) described synthesis and pharmacokinetic properties of nucleoside analogous as possible inhibitors of HCV RNA replication. The authors reported, that 2' - modified nucleosides display high inhibiting activity in investigations on cell replicons. Olsen et al. (Oral Session V, Hepatitis C Virus, Flaviviridae; 16th International Conference on Antiviral Research (Apr. 27, 2003, Savannah, Ga.) p A76) also demonstrated the action of 2'- modified nucleosides in HCV RNA replication.

Nonnucleoside allosteric inhibitors of HIV reverse transcriptase exhibited the proved effective therapeutic action both by themselves and in combination with nucleoside inhibitors and with protease inhibitors. Several classes of nonnucleoside HCV NS5B inhibitors have already been described and are at present at the stage of trials including benzimidazoles (H.Hashimoto et al. WO 01/47833, H.Hashimoto et al. WO 03/000254, P.L.Beaulien et al. WO 03/020240:A2; P.L.Beaulien et al. US 6448281 BI P.L.Beaulien et al. WO 03/007945 A1); indoles (P.L.Beaulien et al. WO 03/0010141 A2); benzothiadiazines, for example 7 (D.Dhanak et al. WO 01/85172 A1; D. Dhanak et al. WO 03/037262 A2; K.J.Duffy et al. WO 03/099801 A1; D.Chai et al. WO 2004052312, D.Chai et al. WO 2004052313, D.Chai et al. WO 02/098424, J.K.Pratt et al. WO 2004/041818 A1; J.K.Pratt et al. WO 2004/087577 A1), tiophenes, for example 8 (C.K.Chan et al. WO 02/100851)

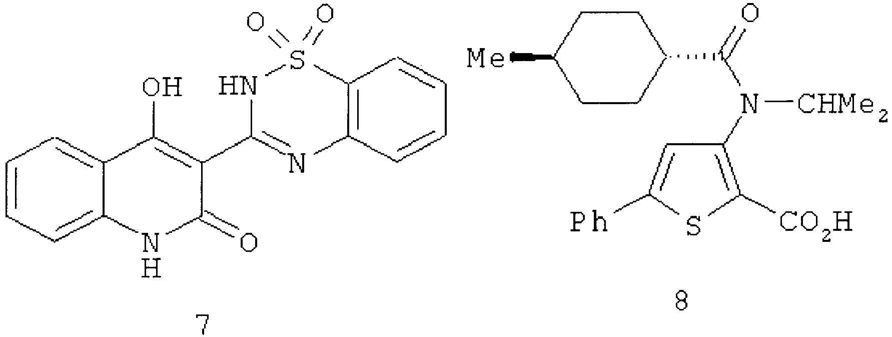

Benzothiophenes (D.C.Young and T.R.Bailey WO 00/18231); β-ketopyruvates (S.Attamura et al. US 6492423 B1, A.Attamura et al. WO 00/06529); pyrimidines (C.Gardelli et al. WO 02/06246 A1); pyrimidindiones (T.R.Bailey and D.C.Young WO 00/13708); triazines (K.-H. Chung et al. WO 02/079187 A1); rhodanine derivatives (T.R.Bailey and D.C.Young WO 00/10573, J.C.Jean et al. WO 01/77091 A2); 2,4-dioxopyranes (R.A.Love et al. EP 256628 A2); phenylalanine derivatives (M.Wang et al. J.Biol. Chem., 2003 278:2489-2495).

Concomitant therapy at which very persistent mutant strains could be suppressed is now a conventional approach in antiviral chemotherapy. Nucleoside inhibitors described here could be combined with other nucleoside inhibitors of HCV polymerase, nonnucleoside inhibitors of HCV polymerase and inhibitors of HCV protease. As emerged and are being developed other classes of drugs against HCV, for example, inhibitors of virus infiltration, helicase inhibitors, IRES inhibitors, ribozymes and antisense oligonucleotides, they can also be excellent candidates for use in concomitant therapy. Interferon derivatives have already successfully combined with Ribavirin, and interferon and chemically modified interferones will be useful in combination with described here nucleosides.

Nucleoside derivatives often represent effective antiviral (for example, HIV, HCV, Herpes simplex, CMV) and anticancer chemotherapeutical remedies. Unfortunately their practical usage is often restricted by two factors. First of all, poor pharmacokinetic properties often restrict nucleoside absorption from gastrointestinal tract and also intercellular concentration of nucleoside derivatives, and secondly suboptimal physical properties restrict the choice of a medical composition, which could be used for increasement of active ingredient liberation.

Up to now hepatitis C is a serious problem of Health Care. It leads to chronic liver disease developing into hepatic cirrhosis and hepatocellular carcinoma.

In this context, now the search for new highly effective anti flaviviral drugs is still one of the main directions in new pharmacological agents creation for treatment of wide and diverse range of viral infections, including HCV. Therefore, up to now synthesis of novel compounds and their use as antiviral active components for pharmaceutical compositions and medicaments, among them HCV, is actual.

### Disclosure of the invention

In the context of this invention, terms are generally defined as follows:
**"Azaheterocycle"** means an aromatic or saturated mono- or polycyclic system comprising at least one nitrogen atom.
**"Alkyl"** means an aliphatic hydrocarbon straight or branched group with 1-12 C-atoms in chain. Branched means that alkyl chain has one or more "lower alkyl" (C₁-C₆)alkyl substituents. The preferred alkyl groups are lower (C₁-C₆) alkyl or methyl, ethyl, *n*-propyl, *iso*-propyl, *n*-butyl, *iso*-butyl, *sec*-butyl, *tert*-butyl, cyclopropylmethyl, cyclobutylmethyl, cyclopentylmethyl, *n*-pentyl, 2-pentyl, 3-pentyl, neo-pentyl, *n*-hexyl, cyclohexyl. Alkyl may have substituents.
**"Alkyloxy (alkoxy)"** means **AlkylO-** group in which alkyl is defined in this section. The preferred alkyloxy groups are methoxy, ethoxy, *n*-propoxy, *iso*-propoxy, *n-*butoxy and *tert*-butoxy.
**"Amino group"** means **R'R"N**-group substituted or not with optionally the same substituents R' and R". Amino group may have substituents.
**"Aminocarbonyl"** means C(=O)N Rₖ^{a}Rₖ₊₁^{a} - group substituted or not substituted with optionally the same carbomoyl substituents Rₖ^{a} and Rₖ₊₁^{a} including hydrogen, methyl, cycloalkyl, which together with N-atom forms pyrrolidine cycle.
**"Aryl"** means an aromatic mono- or polycyclic system with 6-14 carbon atoms, predominantly 6-10 carbon atoms. Aryl may have one or more "cyclic system substituents" of the same or different structure.
**"Aromatic cycle" (aromatic system)** means a plane cyclic system, in which all atoms take part in the formation of a common conjugation system comprising, according to Hückel rule, (4n + 2) π-electrons (n is a whole nonnegative number). Benzene, naphthalene, anthracene and others are the representatives of aromatic cycles. In the case of "heteroaromatic cycles" π-electrons and p-electrons of heteroatoms participate in the conjugation, so that their total number is equal to (4n + 2) as well. Pyridine, thiophene, pyrrole, furan, thiazole and others are the examples of such cycles. Aromatic cycle may have one or more "cyclic system substituents" and could be annelated to nonaromatic cycle, heteroaromatic or heterocyclic system.
**"Acyl group" (Acyl)** means R-C(=O)-, (preferably C₁-C₆acyl), optionally substituted C₁-C₅alkyl-C(=O)-, C₁-C₅alkenyl-C(=O)-, C₁-C₅cycloalkyl-C(=O)-, (preferably cyclopropyl-C(=O)-, cyclobutyl-C(=O)-); heterocyclyl-C(=O)-, (preferably 2-methylfuran), aryl-C(=O)- (aroyl), aralkyl-C(=O)-, (preferably 3-phenylpentane-C(=O)-), heteroaryl-C(=O)- (heteroaroyl), heteroarylalkyl-C(=O)-groups, in which C₁-C₅alkyl-, C₁-C₅alkenyl-, C₁-C₅cycloalkyl-, heterocyclyl-, aryl-, aralkyl-, heteroaryl-, heteroarylalkyl-, methoxy groups, the mentioned groups may have substituents, see "cyclic system substituents", "substituted alkyl", "substituted alkenyl", "heterocyclic system substrituents" which are defined in this section.
**"α-aminoacyl group" (α-aminoacyl)** means -C(=O)CH(R²ₖ)NR²ₖ₊₁R²ₖ₊₂, where R²ₖ, R²ₖ₊₁ and R²ₖ₊₂ may take on a value hydrogen, C₁-C₅alkyl-, aryl, heterocyclyl, or NR²ₖR²ₖ₊₂ represents 5-6 membered heterocycle, the mentioned groups may have substituents, see "cycle system substituents", "substituted alkyl", "substituted alkenyl", "heterocyclic system substituents", "substituted aryl", defined in this section.
**"Active component"** (drug-substance) means a physiologically active compound of synthetic or other (biotechnological, vegetable, animal, microbe and so on) origins exhibiting pharmacological activity which is an active ingredient of pharmaceutical composition employing in production and preparation of medicaments.
**"Heteroaryl" (hetaryl)** means aromatic monocyclic or polycyclic system, including from 5 to 14 carbon atoms, preferably from 5 to 10, in which one or moce carbon atoms are substituted with heteroatom or heteroatoms such as nitrogen, sulfur or oxygen. Prefix "aza", "oxa" or "thia" before "heteroaryl" means the presence of atom nitrogen, oxygen or sulfur, respectively in the cyclic system. N-atom in heteroaryl could be oxidized to N-oxide. Heteroaryl may have one or more "cyclic system substituent" which could be identical or different. The preferable heteroaryl are pyrrolyl, furanyl, thienyl, pyridinyl, pyrrolidine, imidazolyl, oxazolyl, benzothiadiazole, indolyl, azaindolyl, benzodiazolyl, benzothiazolyl, quinolynyl, imidazolyl, thienopyridyl, quinazolynyl, thenopyrimidinyl, pyrrolopyridinyl, imidazopyridyl, isoquinolynyl, benzoazaindolyl, 1,2,4-triazynyl, thienopyrrolyl, furopyrrolyl and others.
**"Heterocyclyl"** means an aromatic or nonaromatic saturated or partially saturated mono- or polycyclic system with 3-10 carbon atoms, preferably from 4 to 6, wherein one or more carbon atoms are substituted by one or more heteroatoms, such as N, O, S or P. Prefix "aza", "oxa" or "thia" before "heterocyclyl" means that N, O or S atoms are introduced in the cycle, respectively. Heterocyclyl may have one or more substituents of the same or different structure. N- and S- atoms of heterocyclyl could be oxidized to N-oxide, S-oxide or S-dioxide. Piperidinyl, pyrrolidinyl, piperazinyl, morpholinyl, thiomorpholinyl, thiazolidinyl, 1,4-dioxan-2-yl, tetrahydrofuranyl, tetrahydrothiophenyl and others are examples of heterocyclyl.
**"Substituted alkenyl"** substituted alkenyl may also have one or more identical or different substituents, including halogen, alkenyloxy, aroyl, heteroaroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonyl, heteroaralkyloxy and others. The preferable alkenyl groups are ethenyl, propenyl, *n*-butenyl, *iso*-butenyl, 3-methylbut-2-enyl, *n*-pentenyl and *n*-hexenyl.
**"Substituted alkyl"** substituted alkyl may have one or more identical or different substituents, including halogen, alkenyloxy, cycloalkyl, aryl, heteroaryl, heterocyclyl, aroyl, heteroaroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonyl, heteroaralkyloxy Rₖ^{a}Rₖ₊₁^{a}N-, where Rₖ^{a} and Rₖ₊₁^{a} independently of each other represent "amino group substituents", the meanings of which are defined in this section, for example, hydrogen, alkyl, aryl, aralkyl, heteroaralkyl, heterocyclyl or heteroaryl, or Rₖ^{a} and Rₖ₊₁^{a} together with the N-atom, they are attached to form through Rₖ^{a} and Rₖ₊₁^{a} 4-7 membered heterocyclyl or heterocyclenyl. The preferable "alkyl substituents" are aryl, heteroaryl, heterocyclyl, hydroxy, C₁-C₅ alkoxy, C₁-C₅ alkoxycarbonyl, aralkoxy, aryloxy, alkylthio, heteroarylthio, aralkylthio, alkylsufonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊ᵢ^{a}NC(=O)-, annelated arylheterocyclenyl, annelated arylheterocyclyl. That is: hydroxy group; substituted with fluorophenyl oxy group, mono- and diC₁-C₅alkylamino group (dimethylamino group, methylamino group), C₁-C₅alkyloxycarbonyl group (ethoxycarbonyl), phenyl, chlorophenyl, fluorophenyl, C₁-C₅alkyl (methyl) substituted phenyl, C₁-C₅cycloalkyl (cyclopentyl) substituted phenyl, C₁-C₅alkoxy substituted phenyl (methoxyphenyl), thiophenyl, furanyl, pyrrolidine.
**"Amino group substituents"** amino group substituents R' and R" represent hydrogen, optionally substituted C₁-C₅alkyl, optionally substituted C₁-C₅cycloalkyl (see cyclic system substituent), optionally substituted aryl (see cyclic system substituent), optionally substituted heteroaryl (see cyclic system substituent), optionally substituted heterocyclyl (see cyclic system substituent), C₁-C₅alkenyl, acyl, aroyl, heteroaroyl, C₁-C₅alkylsulfonyl, arylsulfonyl, heteroarylsulfonyl, alkoxycarbonyl substituted with linear or branched C₁-C₅alkyl, halogen, heterocyclyl; aryloxycarbonyl, aralkoxycarbonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, optionally substituted aminosulfonyl. **R'R"N**-Group may represent nonaromatic azaheterocycle, preferably azetidine, pyrrolidine, piperidine, morpholine, thiomorpholine, piperazine, homopiperidine, homopiperazine. The preferable amino group substituents are hydrogen, C₁-C₅alkyl, C₁-C₅cycloalkyl, substituted C₁-C₅alkyl ("substituted alkyl"), optionlally simultaneously substituted with 1-3 radicals substituted phenyl (with C₁-C₅alkyl, halogen, C₁-C₅alkoxy group, C₁-C₅alkyloxycarbonyl), pyridinyl, optionally substituted thiophenyl, optionally substituted furanyl (see cyclic system substituents).
**"Cyclic system substituents"** could be representatives of aryl groups, prerefably phenyl or naphthyl, substituted phenyl or substituted naphthyl. Aryl could be annelated with a nonaromatic cycle system or heterocycle. The preferable cycle system substituents are hydrogen, halogens (chloro, fluoro, bromo), optionally substituted C₁-C₅alkyl, optionally substituted cycloC₁-C₅alkyl, C₁-C₅alken, hydroxy group, C₁-C₅alkyloxy group (methoxy, ethoxy, propoxy, diester of ethylene glycol, diester of methane diol), cyano group, C₁-C₅alkyloxycarbonyl (methyl, ethyl), alkylthio group (methylthio), carboxy group, aminocarbonyl (see "aminocarbonyl"), phenyl annelated with 5-7 membered saturated cycle including 1-3 heteroatoms (preferably nitrogen, oxygen and sulfur atoms).
**"Substituent"** means a chemical radical attached to a scaffold (fragment), for example, "alkyl substituent", "amino group substituent", "carbamoyl substituent", and "cyclic system substituent", the meanings of which are defined in this section.
**"Substituted aminocarbonyl group" (aminocarbonyl)** means **R'R"N-C(=O)-**group, in which the substituents R' and R" could be represented by optionally substituted alkyl, alkenyl, alkynyl, cycloalkyl, aryl, heteroaryl, and heterocyclyl, the meanings of which are defined in this section. The preferred aminocarbonyl groups are optionally substituted C₁-C₅alkyl, C₁-C₅alkenyl, C₁-C₅cycloalkyl, optionally substituted aryl (see cyclic system substituent), optionally substituted hetaryl (see cyclic system substituent), optionally substituted heterocyclyl (see heterocyclyl substituent) or amino group **R'R"N.**
**"Substituted oxycarbonyl group" (oxycarbonyl)** means **R-O-C(=O)-** group, in which the substituent R could be represented by optionally substituted alkyl, alkenyl, cycloalkyl, aryl, heteroaryl and heterocyclyl, the meanings of which are defined in this section. The preferable oxycarbonyl groups are methoxycarbonyl, ethoxycarbonyl, *tert*-butoxycarbonyl, and benzyloxycarbonyl.
**"Carbamoyl substituent"** means a substituent attached to aminocarbonyl group, the meanings of which are defined in this section. Carbamoyl substituent represents hydrogen, alkyl, cycloalkyl, aryl, heteroaryl, heterocyclyl, alkoxycarbonylalkyl, aralkoxycarbonylalkyl, heteroaralkyloxy-carbonylalkyl or Rₖ^{a}Rₖ₊₁^{a}N-, Rₖ^{a}Rₖ₊₁^{a}NC(=O)-alkyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl. The preferable "carbamoyl substituents" are alkyl, cycloalkyl, aryl, heteroaryl, heterocyclyl, alkyloxycarbonylalkyl, aralkoxycarbonylalkyl, heteroaralkyloxy-carbonylalkyl or Rₖ^{a}Rₖ₊₁^{a}N-, Rₖ^{a}Rₖ₊₁^{a}NC(=O)-alkyl, annelated arylheterocyclenyl, annelated arylheterocyclyl.
**"Heterocycle substituents"** could be representatives of aryl groups, prerefably phenyl or naphthyl, substituted phenyl or substituted naphthyl. Aryl could be annelated with a nonaromatic cycle system or heterocycle. The preferable cycle system substituents are hydrogen, halogens (chloro, fluoro, bromo), optionally substituted C₁-C₅alkyl, optionally substituted cycloC₁-C₅alkyl, C₁-C₅alken, hydroxy group, C₁-C₅alkyloxy group (methoxy, ethoxy, propoxy, diester of ethylene glycol, diester of methane diol), cyano group, C₁-C₅alkyloxycarbonyl (methyl, ethyl), alkylthio group (methylthio), carboxy group, aminocarbonyl (see "aminocarbonyl"), phenyl annelated with 5-7 membered saturated cycle including 1-3 heteroatoms (preferably, nitrogen, oxygen and sulfur atoms).
**"Medicament" -** is a compound (or a mixture of compounds as a pharmaceutical composition) in the form of tablets, capsules, injections, ointments and other ready forms intended for restoration, improvement or modification of physiological functions in humans and animals, and for treatment and prophylaxis of diseases, for diagnostics, anesthesia, contraception, cosmetology and others.
**"Lower alkyl"** means a straight or branched alkyl with 1-6 carbon atoms.
**"Therapeutic kit"** represents a simultaneously administered combination of two or more medicaments exhibiting different mechanism of pharmacological action and directed to various biotargets taking part in disease process.
**"Pharmaceutical composition"** means a composition comprising a compound of the formula I and at least one of the component selected from the group consisting of pharmaceutically acceptable and pharmacologically compatible fillers, solvents, diluents, carriers, auxiliary, distributing and sensing agents, delivery agents, such as preservatives, stabilizers, filler, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the type and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against action of microorganisms can be provided by various antibacterial and antifungal agents, such as, for example, parabens, chlorobutanole, sorbic acid, and similar compounds. Composition may also contain isotonic agents, such as, for example, sugars, sodium chloride, and similar compounds. Prolonged action of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and polyethylene glycol of high molecular weight. Pharmaceutical composition for peroral, sublingval, transdermal, intramuscular, intravenous, subcutaneous, local or rectal administration of active ingredient, alone or in combination with another active compound may be administered to human and animals in a standard administration form, in a mixture with traditional pharmaceutical carriers. Suitable standard administration forms include peroral forms such as tablets, gelatin capsules, pills, powders, granules, chewing-gums and peroral solutions or suspensions; sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. Salts could be prepared in situ in the processes of synthesis, isolation or purification of compounds or they could be prepared specially. In particular, salts of bases specially could be prepared from purified base of the disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, *p*-toluenesulfonates, citrates, maleates, fumarates, succinates, tartrates, methane sulphonates, malonates, salicylates, propionates, ethane sulphonates, benzene sulfonates, sulfamates and the like (Detailed description of such salts properties is given in: Berge S.M., et al., "Pharmaceutical Salts" J.Pharm.Sci., 1977, 66: 1-19). Salts of the disclosed acids may also be prepared by the reaction of purified acids specifically with suitable base; moreover, metal salts and amine salts may be synthesized too. Metal salts are salts of sodium, potassium, calcium, barium, zinc, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of the disclosed acid salts are amines and amino acids of sufficient basicity to produce stable salt and suitable for medical purposes use (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from the main aminoacids - lysine, ornithine and agrinine.

The purpose of the present invention is creating novel inhibitors of RNA polymerase.

The purpose in view is achieved by novel nucleozide inhibitors of RNA polymerase of the general formula **1,** stereoisomers, salts, hydrates, solvates or crystalline forms thereof.

The subject of the present invention is novel substituted alkyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionates of the general formula **1,** stereoisomers, salts, hydrates, solvates or crystalline forms thereof: 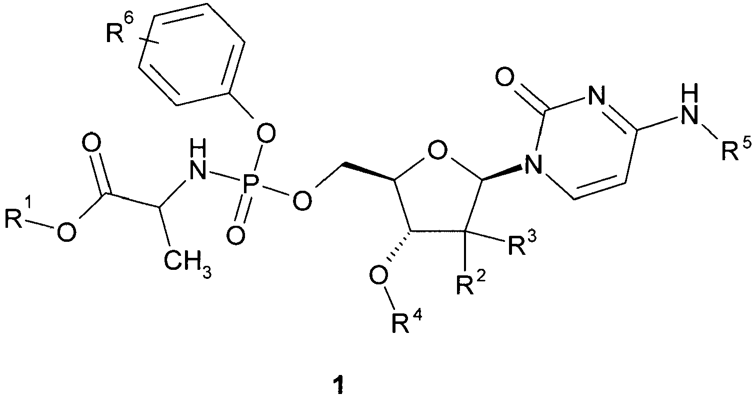 wherein
R¹ represents C₁-C₄alkyl;
R² and R³ represents fluoro, or
R² represents fluoro, and R³ represents methyl;
R⁴ and R⁵ represents H, or
R⁴ represents C₁-C₆acyl, R⁵ represents hydrogen, or
R⁴ represents hydrogen, and R⁵ represents C₁-C₆acyl, or
R⁴ represents optionally substituted α-aminoacyl, and R⁵ represents hydrogen, or
R⁴ represents hydrogen, and R⁵ represents optionally substituted α-aminoacyl;
R⁶ represents hydrogen, methyl, methoxy or halogen.

The preferable are (S)-*iso*-propyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionate of the general formula **2** and (S)-*iso*-propyl 2-{[(2R,3S,5R)-3-acetoxy-5-(4-amino-2-oxo-2H-pyrimidin-1-yl)-tetrahydro-furan-2-ylmethoxy]-phenoxy-phosphorylamino}-propionate of the general formula **3,** stereoisomers, salts, hydrates, solvates or crystalline forms thereof:

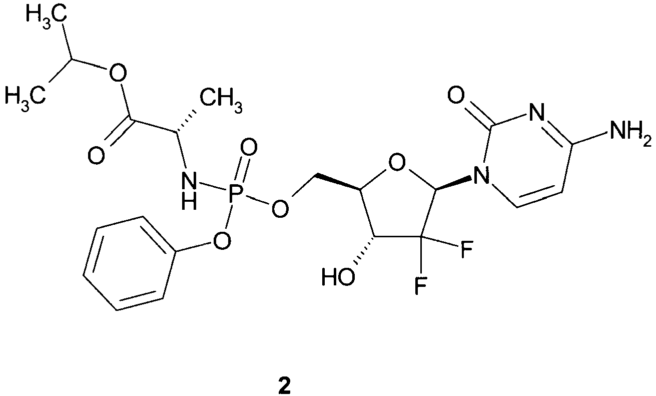

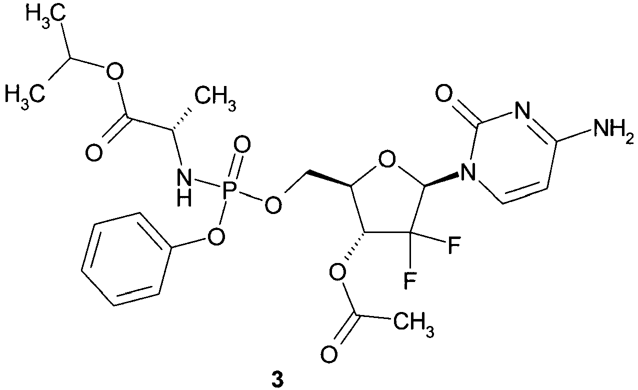

The more preferrable are substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formula **1.1** and substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formula **1.2.** 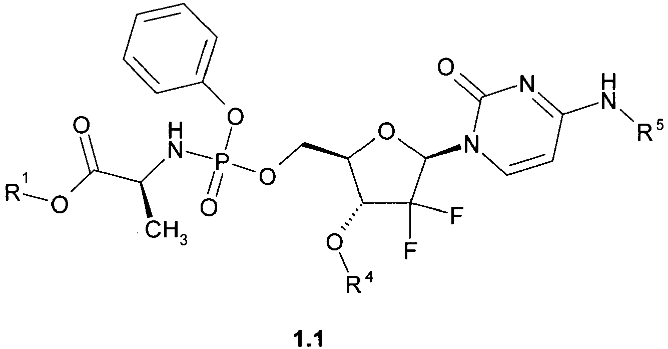 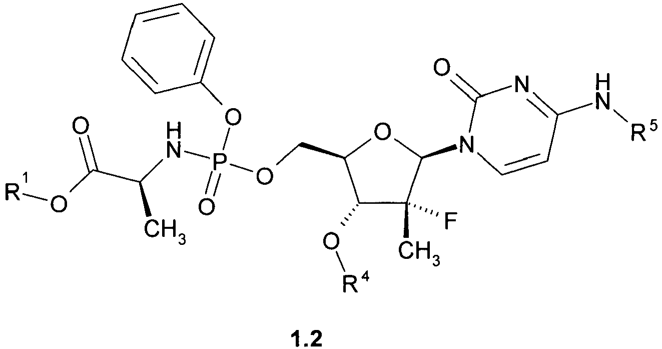 where
R¹, R⁴ and R⁵ have the above meanings.

The more preferrable are substituted alkyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionate, substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formula **1.1.1**, **1.1.2** and substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formulas **1.2.1, 1.2.2.** 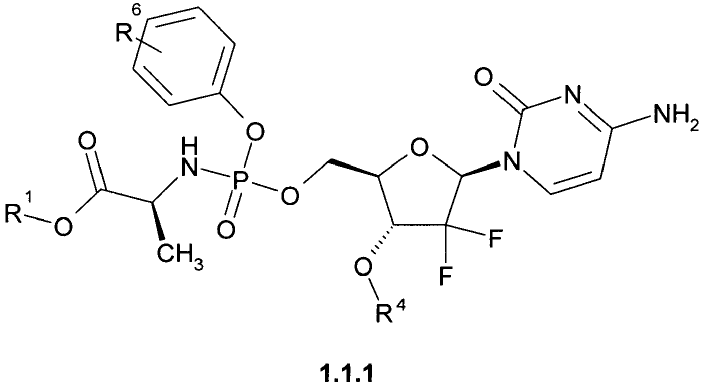 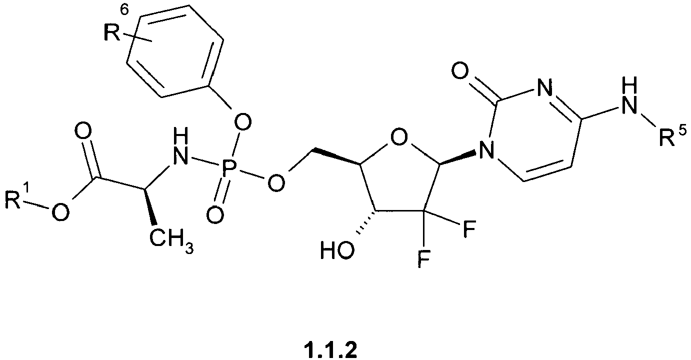 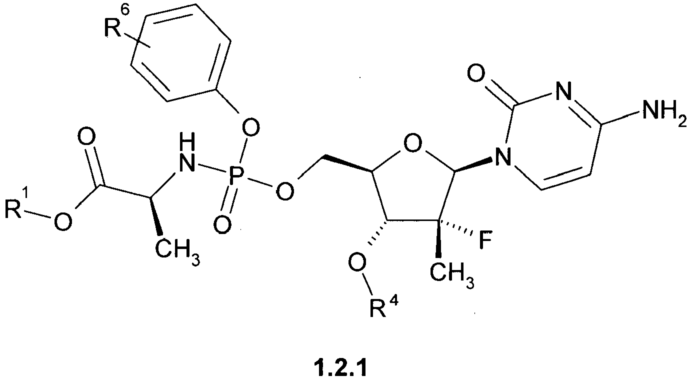 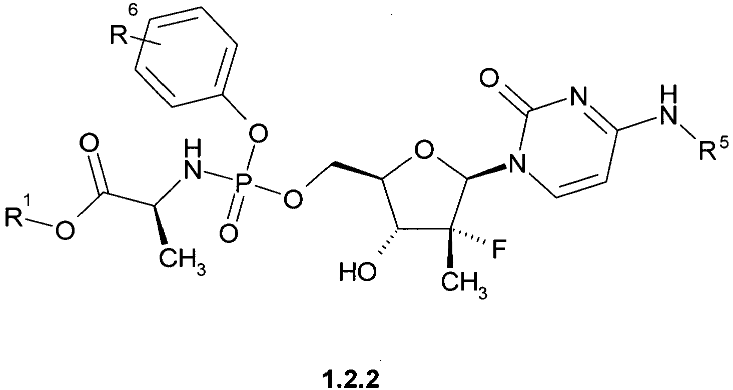 wherein
R¹, R⁴, R⁵ and R⁶ have the above meanings.

The most preferable substituted alkyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionates are:
(*S*)-*iso*-propyl 2-{[(2*R*,3*S*,5*R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1));**
(2R,3S,5R)-5-(4-amino-2-oxo-2H-pyrimidin-1-yl)-4,4-difluoro-2-[((S)-1-*iso*propoxycarbonyl-ethylamino)-phenoxy-phosphorylaminomethyl]-tetrahydro-furan-3-yl (S)-1-methyl-pyrrolidine-2-carboxylate **(1.1.1(2));**
(2R,3R)-5-(4-amino-2-oxo-2H-pyrimidin-1-yl)-4,4-difluoro-2-[(S)-((S)-1-*iso*propoxycarbonyl-ethylamino)-phenoxy-phosphorylaminomethyl]-tetrahydro-furan-3-yl (S)-2-*tert*-butoxycarbonylamino-3-methyl-butanoate **(1.1.1(3));**
(2R,3R)-5-(4-amino-2-oxo-2H-pyrimidin-1-yl)-4,4-difluoro-2-[(R)-((S)-1-isopropoxycarbonyl-ethylamino)-phenoxy-phosphorylaminomethyl]-tetrahydro-furan-3-yl (S)-2-*tert*-butoxycarbonylamino-3-methyl-butanoate **(1.1.1 (4));**
*iso-*propyl (S)-2-({(2R,3R,5R)-5-[4-(2-dimethylamino-acetylamino)-2-oxo-2H-pyrimidin-1-yl]- 4,4-difluoro-3-hydroxy-tetrahydro-furan-2-ylmethoxy]-phenoxy-phosphorylamino}-propionate **(1.1.2(1));**
*iso-*propyl (S)-2-({(2R,3R,5R)-5-[4-((R)-2-*tert*-butoxycarbonylamino-3-methyl-butyrylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(2));**
*iso-*propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy]-phenoxy-phosphorylamino}-propionate **(1.1.2(3));**
methyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)]-phenoxy-phosphorylamino}-propionate **(1.1.2(4));**
*iso-*propyl (R)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-[(4-chlorophenoxy)-((2R,3R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(6));**
*iso-*propyl (S)-2-[(2,4-dichlorophenoxy)-((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-phenoxy-phosphorylamino)-propionate **(1.1.2(7));**
*iso-*propyl (S)-2-[((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-p-tolyl-phosphorylamino)-propionate **(1.1.2(8));**
*iso-*propyl (S)-2-((S)-{(2R,3R,5R)-5-[4-(2-dimethylaminoacetylamino)-2-oxo-2H-pyrimidin-1-yl]-4,4-difluoro-3-hydroxy-tetrahyd ro-furan-2-ylmethoxy)-phenoxy-phosphorylamino)-propionate **(1.1.2(9));**
*iso-*propyl (S)-2-((R)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(10));**
*iso-*propyl (S)-2-[(S)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(11));**
*iso-*propyl (S)-2-[(R)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(12));**
methyl (S)-2-{[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(13));**
*iso-*propyl (S)-2-[((2R,3R,5R)-4,4-difluoro-3-hydroxy-5-{4-[((S)-1-methylpyrrolidine-3-carbonyl)-amino]-2-oxo-2H-pyrimidin-1-yl}-tetrahydro-furan-2-ylmethoxy)-phenoxy-phosphorylamino)-propionate **(1.1.2(14));**
*iso-*propyl (S)-2-[((2R,3R,5R)-4,4-difluoro-3-hydroxy-5-{4-[((S)-1-methylpyrrolidine-4-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy- phosphorylamino)-propionate **(1.2.2(1));**
*iso-*propyl (R)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(2));**
*iso-*propyl (R)-2-[((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(3));**
*iso-*propyl (S)-2-((S)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(4));**
*iso-*propyl (S)-2-((R)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(5));**
*iso-*propyl (S)-2-[(S)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(6));**
*iso-*propyl (S)-2-[(R)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(7)).**

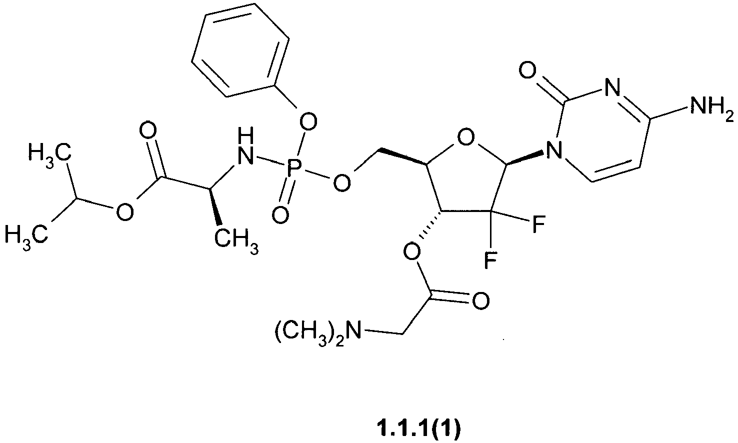

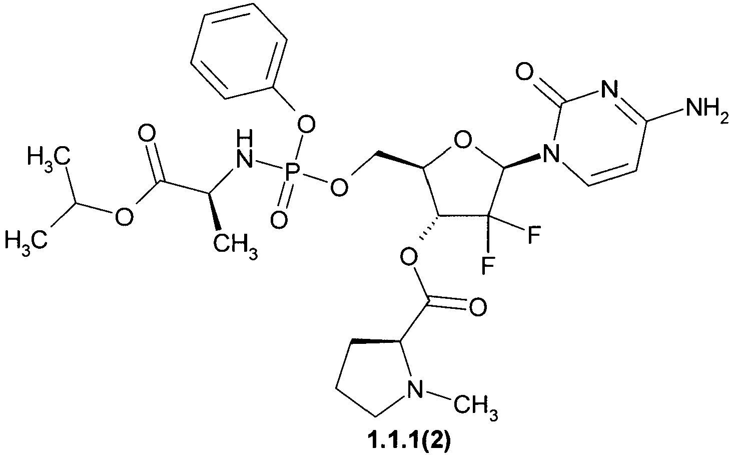

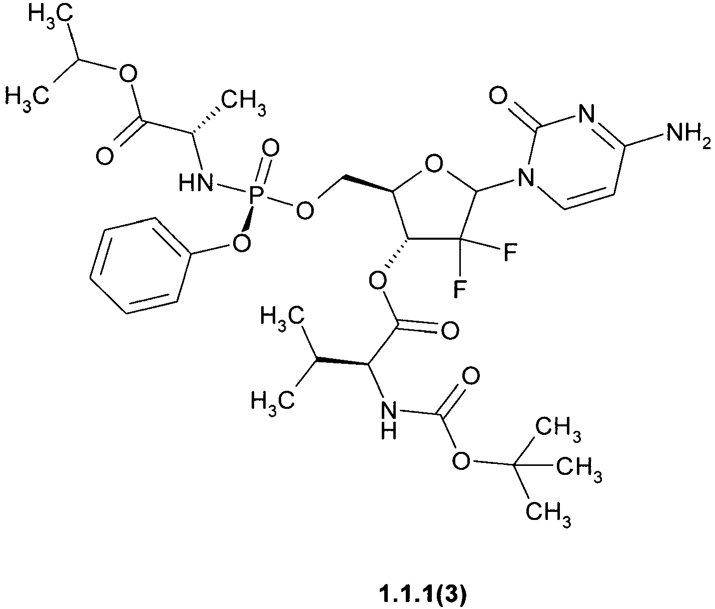

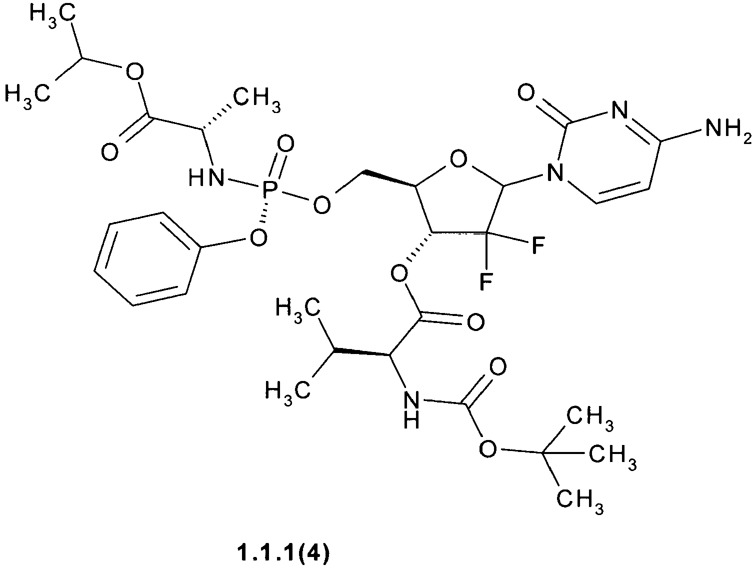

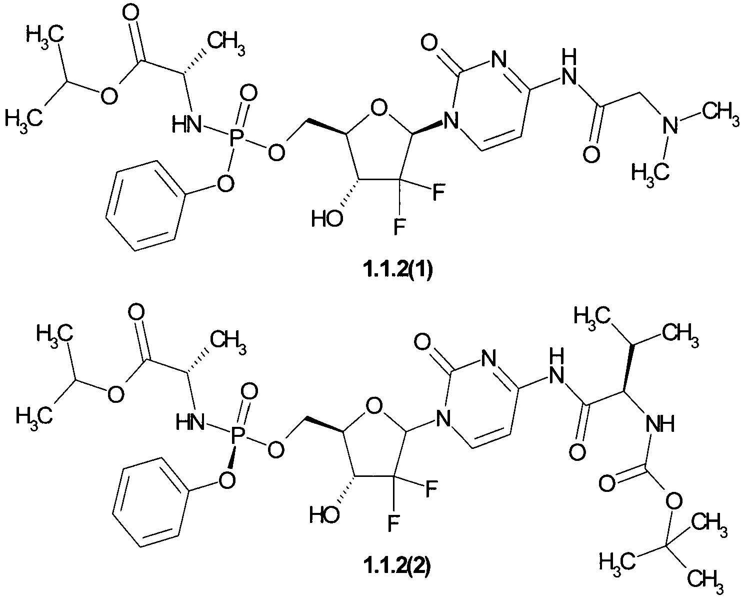

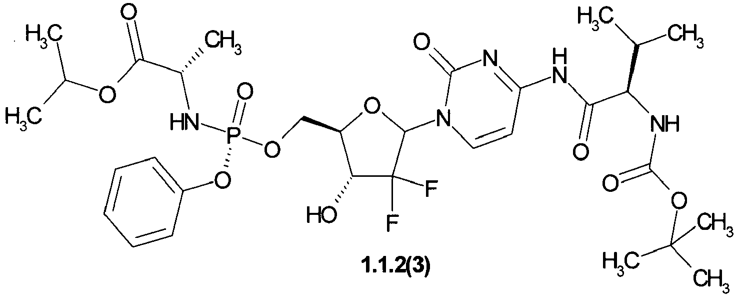

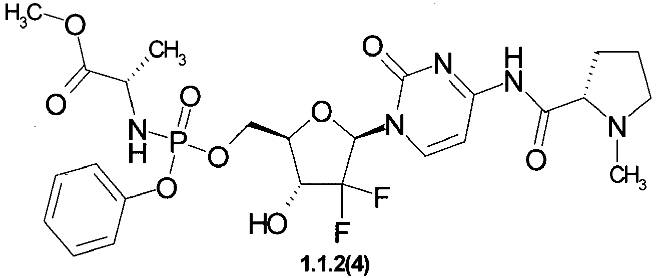

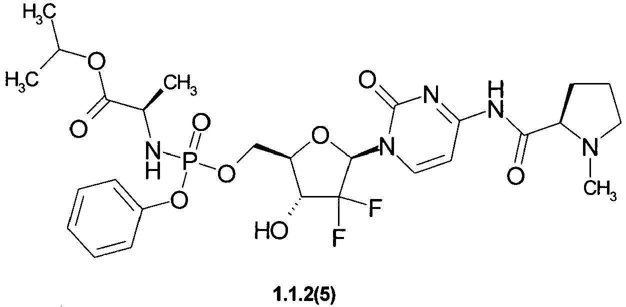

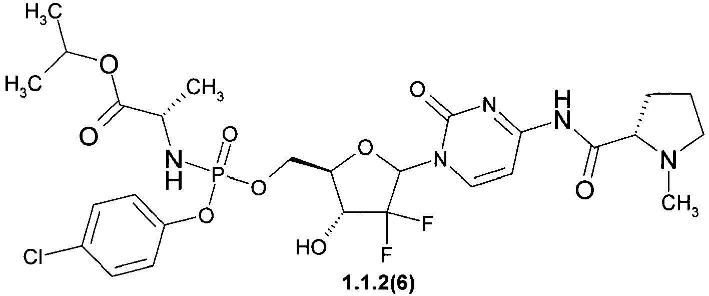

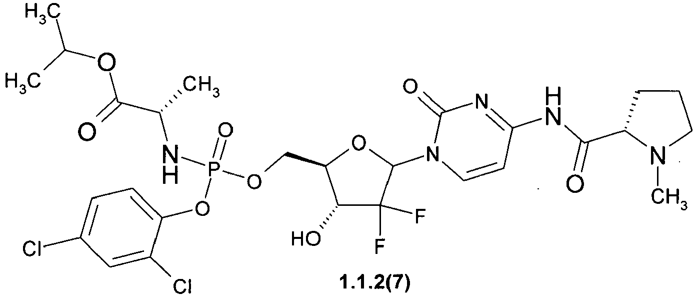

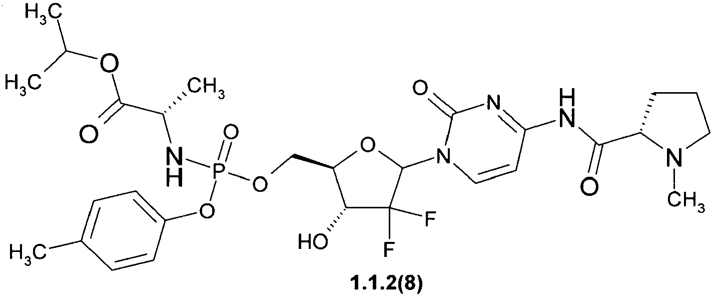

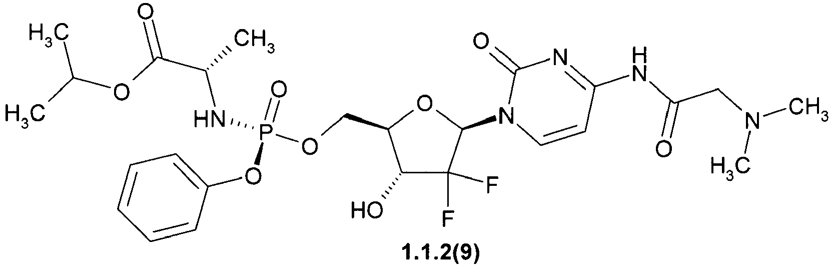

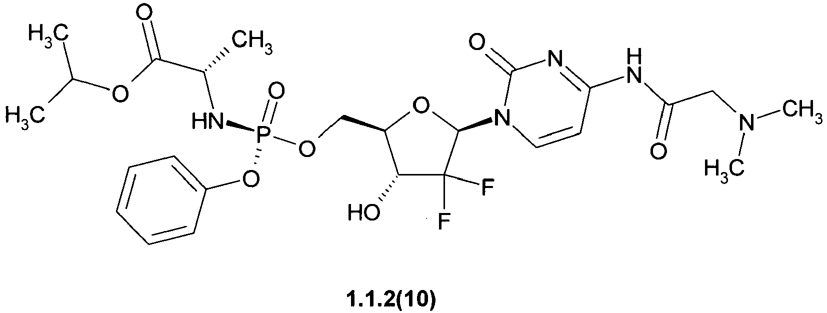

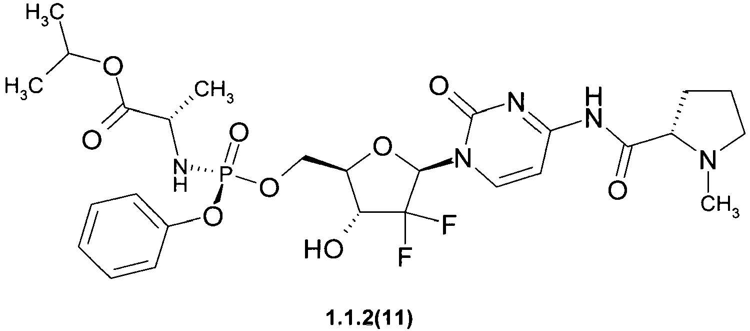

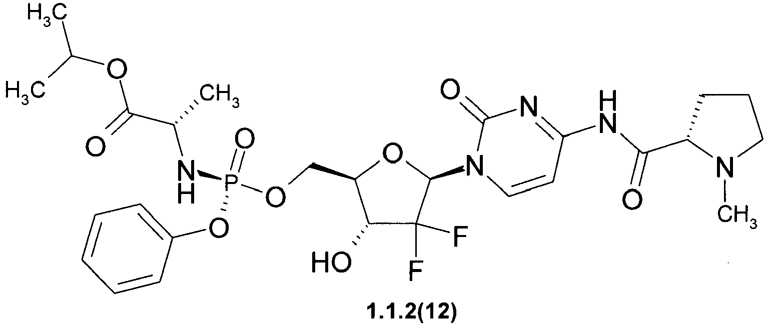

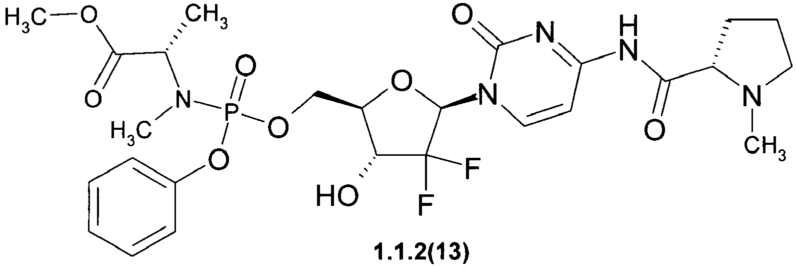

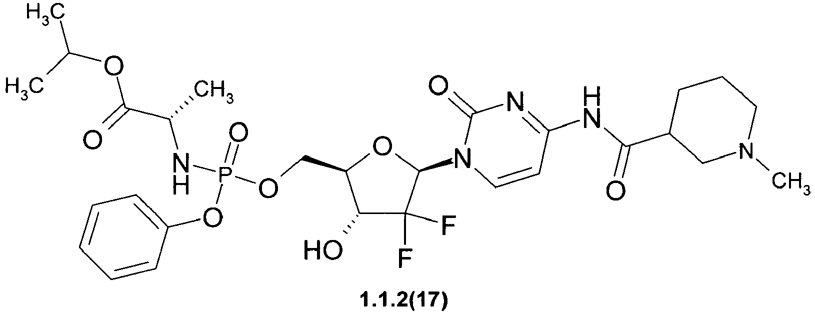

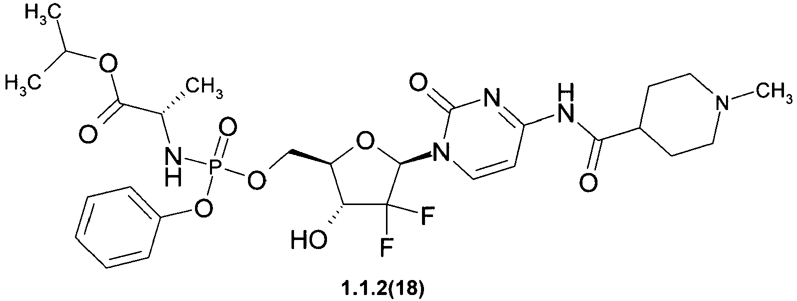

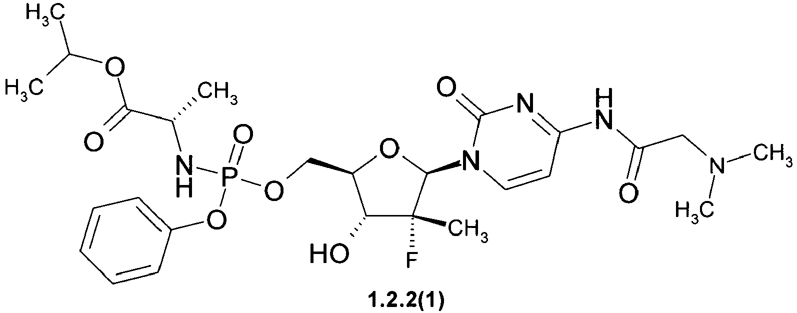

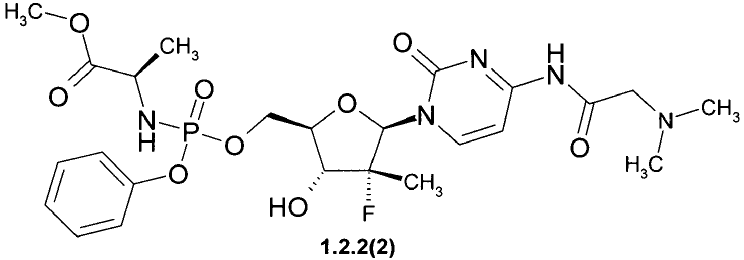

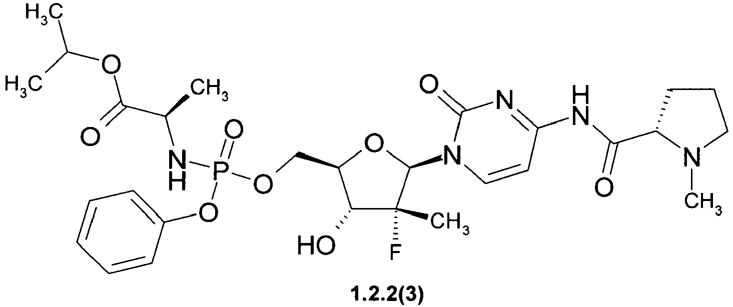

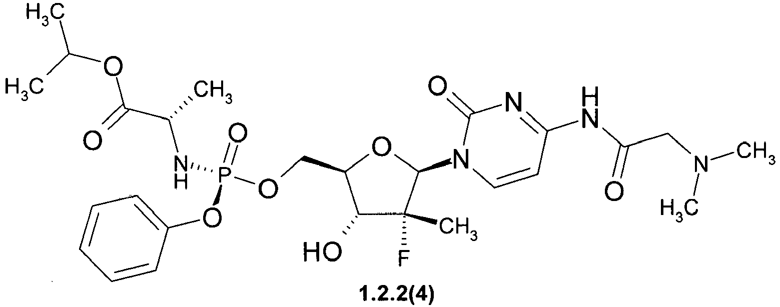

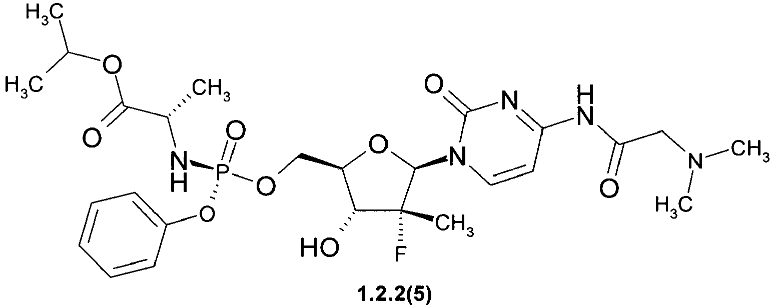

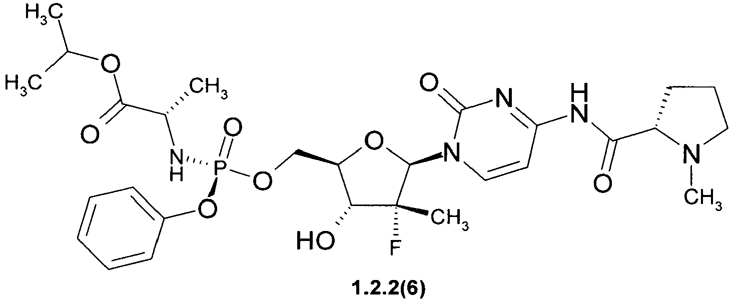

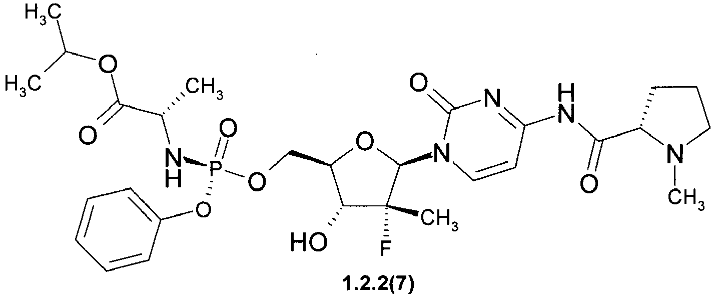

The subject of the present invention is a method for preparation of compounds of the general formula **1** and the general formula **2,** stereoisomers, pharmaceutically acceptable salts, hydrates, solvates or crystalline forms thereof, by interaction of a compound of general formula **4** with a compound of general formula **5** 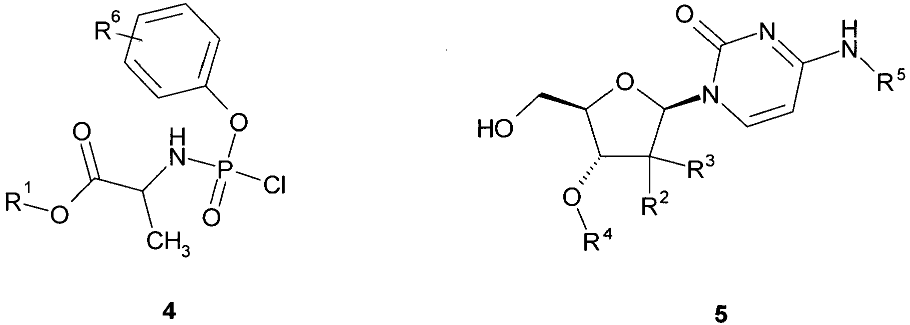 wherein
R¹, R², R³, R⁴, R⁵ and R⁶ have the above meanings.

The subject of the present invention is a method for preparation of compound of general formula **2,** stereoisomers, salts, hydrates, solvates or crystalline forms thereof, by interaction of a compound of the general formula **4(1)** with a compound of a general formula **5(1).** 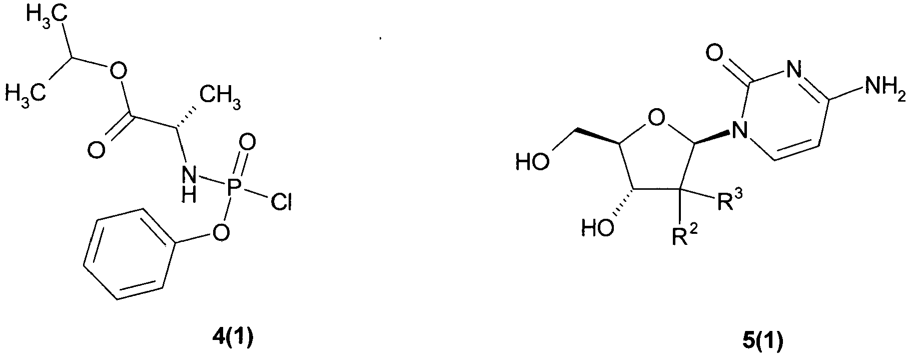 where
R², R³ have the above meanings.

The subject of the present invention is a method for preparation of compound of the general formula **1,** stereoisomers, pharmaceutically acceptable salts, hydrates, solvates or crystalline forms thereof, by interaction of a compound of the general formula **6** with an anhydride of the general formula **7** in presence of 4-dimethylaminopyridine (DMAP) and triethylamine.

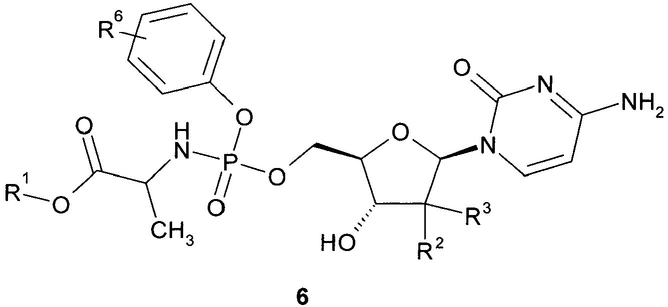

(R⁴)²O 7

wherein
R⁴ represents optionally substituted α-aminoacyl, and R⁵ represents hydrogen,
R¹, R², R³ and R⁶ have the above meanings.

The subject of the present invention is a method for preparation of compound of the general formula **3,** stereoisomers, salts, hydrates, solvates or crystalline forms thereof, by interaction of a compound of the general formula **2** with acetic anhydride in presence of 4-dimethylaminopyridine (DMAP) and triethylamine.

The subject of the present invention is a method for preparation of compounds of the general formula **1,** stereoisomers, pharmaceutically acceptable salts, hydrates, solvates or crystalline forms thereof, by interaction of a compound of the general formula **6** with α-amino acid of the general formula **8** in presence of 1,1'-carbonyldiimidazole.

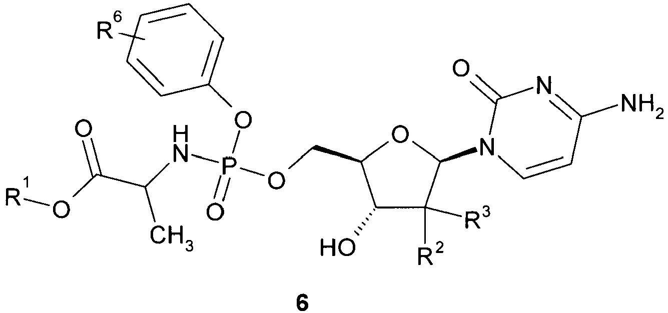

R⁵-OH 8

wherein
R⁴ represents hydrogen, and R⁵ represents optionally substituted α-aminoacyl, and
R¹, R², R³ and R⁶ have the above meanings.

The subject of the present invention is a method for preparation of compounds of the general formula **1,** stereoisomers, salts, hydrates, solvates or crystalline forms thereof, by interaction of a compound of the general formula **9** with an acid of the general formula **10** in presence of 1,1'-carbonyldiimidazole and subsequent removal of Boc-protecting group in the resulting product **11.**

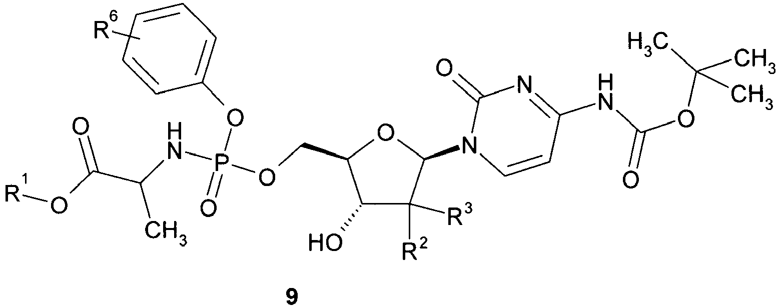

R⁴-OH 10

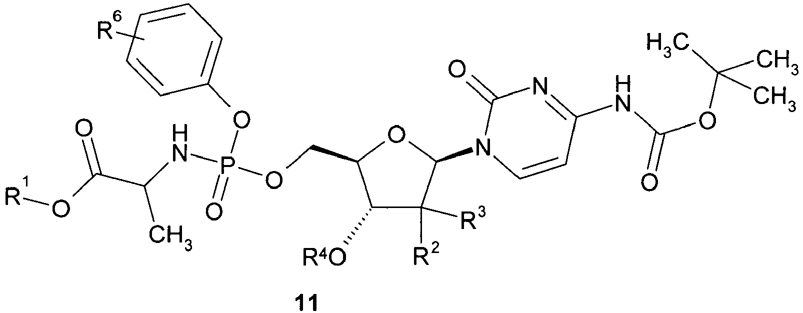

wherein
R⁴ represents optionally substituted α-aminoacyl, and
R⁵ represents hydrogen, and
R¹, R², R³ and R⁶ have the above meanings.

Resolution of compounds of the general formulas **1, 2** and **3** into their phosphorous stereoisomers is carried out by crystallization and/or by highpressure liquid chromatography (HPLC).

The starting compounds used for preparation of novel inhibitors of the general formulas **1, 2** and **3** are commercially available or were prepared by known methods described in literature.

The subject of the present invention is an active component exhibiting properties of nucleoside inhibitor of HCV RNA polymerase NS5B representing substituted alkyl 2-{[(2R,3S,5R)-5-(4-amino-2-oxo-2H-pyrimidin-1-yl)-3-hydroxy-tetrahydrofuran-2-ylmethoxy]-phenoxy-phosphorylamino}-propionate of the general formula **1,** its stereisomer, salt, hydrate, solvate or crystalline form, (S)-*iso*-propyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionate of the general formula **2** and (*S*)-*iso*-propyl 2-{[(2R,3S,5R)-3-acetoxy-5-(4-amino-2-oxo-2H-pyrimidin-1-yl)-tetrahydro-furan-2-ylmethoxy]-phenoxy-phosphorylamino}-propionate of the general formula **3,** its stereoisomers, salts, hydrates, solvates or crystalline forms.

The more preferred nucleoside inhibitor of HCV RNA polymerase NS5B is substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formula **1.1** and substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formula **1.2.**

The more preferred nucleoside inhibitor of HCV RNA polymerase NS5B is also substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formulas **1.1.1, 1.1.2** and substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formulas **1.2.1, 1.2.2.**

The most preferrable nucleoside inhibitor of HCV RNA polymerase NS5B is:
(*S*)-*iso*-propyl 2-{[(2*R*,3*S*,5*R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1));**
(2R,3S,5R)-5-(4-amino-2-oxo-2H-pyrimidin-1-yl)-4,4-difluoro-2-[((S)-1-*iso*propoxycarbonyl-ethylamino)-phenoxy-phosphorylaminomethyl]-tetrahydro-furan-3-yl (S)-1-methyl-pyrrolidine-2-carboxylate **(1.1.1(2));**
(2R,3R)-5-(4-amino-2-oxo-2H-pyrimidin-1-yl)-4,4-difluoro-2-[(S)-((S)-1-*iso-*propoxycarbonyl-ethylamino)-phenoxy-phosphorylaminomethyl]-tetrahydro-furan-3-yl (S)-2-*tert*-butoxycarbonylamino-3-methyl-butanoate **(1.1.1 (3));**
(2R,3R)-5-(4-amino-2-oxo-2H-pyrimidin-1-yl)-4,4-difluoro-2-[(R)-((S)-1-isopropoxycarbonyl-ethylamino)-phenoxy-phosphorylaminomethyl]-tetrahydro-furan-3-yl (S)-2-*tert*-butoxycarbonylamino-3-methyl-butanoate **(1.1.1(4));**
*iso-*propyl (S)-2-({(2R,3R,5R)-5-[4-(2-dimethylamino-acetylamino)-2-oxo-2H-pyrimidin-1-yl]- 4,4-difluoro-3-hydroxy-tetrahydro-furan-2-ylmethoxy]-phenoxy-phosphorylamino}-propionate **(1.1.2(1));**
*iso-*propyl (S)-2-({(2R,3R,5R)-5-[4-((R)-2-*tert*-butoxycarbonylamino-3-methyl-butyrylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(2));**
*iso-*propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy]-phenoxy-phosphorylamino}-propionate **(1.1.2(3));**
methyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(4));**
*iso-*propyl (R)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-[(4-chlorophenoxy)-((2R,3R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(6));**
*iso-*propyl (S)-2-[(2,4-dichlorophenoxy)-((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-phenoxy-phosphorylamino)-propionate **(1.1.2(7));**
*iso-*propyl (S)-2-[((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-*p*-tolylphosphorylamino)-propionate **(1.1.2(8));**
*iso-*propyl (S)-2-((S)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(9));**
*iso-*propyl (S)-2-((R)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(10));**
*iso-*propyl (S)-2-[(S)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(11));**
*iso-*propyl (S)-2-[(R)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(12));**
methyl (S)-2-{[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(13));**
*iso-*propyl (S)-2-[((2R,3R,5R)-4,4-difluoro-3-hydroxy-5-{4-[((S)-1-methylpyrrolidine-3-carbonyl)-amino]-2-oxo-2H-pyrimidin-1-yl}-tetrahydro-furan-2-ylmethoxy)-phenoxy-phosphorylamino)-propionate **(1.1.2(14));**
*iso-*propyl (S)-2-[((2R,3R,5R)-4,4-difluoro-3-hydroxy-5-{4-[((S)-1-methylpyrrolidine-4-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy- phosphorylamino)-propionate **(1.2.2(1));**
*iso-*propyl (R)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(2));**
*iso-*propyl (R)-2-[((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(3));**
*iso-*propyl (S)-2-((S)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(4));**
*iso-*propyl (S)-2-((R)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(5));**
*iso-*propyl (S)-2-[(S)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(6));**
*iso-*propyl (S)-2-[(R)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(7)).**

The subject of the present invention is a method for inhibition of HCV RNA polymerase NS5B, which consists in contacting of HCV RNA polymerase NS5B with substituted alkyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionates of the general formula **1,** or (*S*)-*iso*-propyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionate of the general formula **2** or (*S*)-*iso*-propyl 2-{[(2R,3S,5R)-3-acetoxy-5-(4-amino-2-oxo-2H-pyrimidin-1-yl)-tetrahydro-furan-2-ylmethoxy]-phenoxy-phosphorylamino}-propionate of the general formula **3,** or stereoisomers, salt, hydrate, solvate or crystalline form thereof.

The more preferable method for inhibition of HCV RNA polymerase NS5B is a method, which consists in contacting of HCV RNA polymerase NS5B with substituted alkyl (S)- 2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionates of the general formula **1.1** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionates of the general formula **1.2.**

The more preferable method for inhibition of HCV RNA polymerase NS5B is also a method, which consists in contacting of HCV RNA polymerase NS5B with substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionates of the general formulas **1.1.1, 1.1.2** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionates of the general formulas **1.2.1, 1.2.2.**

The most preferable method for inhibition of HCV RNA polymerase NS5B is a method, which consists in contacting of HCV RNA polymerase NS5B with a compound selected from:
(*S*)-*iso*-propyl 2-{[(2*R*,3*S*,5*R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1));**
(2R,3S,5R)-5-(4-amino-2-oxo-2H-pyrimidin-1-yl)-4,4-difluoro-2-[((S)-1-*iso-*propoxycarbonyl-ethylamino)-phenoxy-phosphorylaminomethyl]-tetrahydro-furan-3-yl (S)-1-methyl-pyrrolidine-2-carboxylate **(1.1.1(2));**
(2R,3R)-5-(4-amino-2-oxo-2H-pyrimidin-1-yl)-4,4-difluoro-2-[(S)-((S)-1-*iso*propoxycarbonyl-ethylamino)-phenoxy-phosphorylaminomethyl]-tetrahydro-furan-3-yl (S)-2-*tert*-butoxycarbonylamino-3-methyl-butanoate **(1.1.1 (3));**
(2R,3R)-5-(4-amino-2-oxo-2H-pyrimidin-1-yl)-4,4-difluoro-2-[(R)-((S)-1-isopropoxycarbonyl-ethylamino)-phenoxy-phosphorylaminomethyl]-tetrahydro-furan-3-yl (S)-2-*tert*-butoxycarbonylamino-3-methylbutanoate **(1.1.1(4));**
*iso-*propyl (S)-2-({(2R,3R,5R)-5-[4-(2-dimethylamino-acetylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(1));**
*iso-*propyl (S)-2-({(2R,3R,5R)-5-[4-((R)-2-*tert*-butoxycarbonylamino-3-methyl-butyrylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(2));**
*iso-*propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy]-phenoxy-phosphorylamino}-propionate **(1.1.2(3));**
methyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)]-phenoxy-phosphorylamino}-propionate **(1.1.2(4));**
*iso-*propyl (R)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-[(4-chlorophenoxy)-((2R,3R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(6));**
*iso-*propyl (S)-2-[(2,4-dichlorophenoxy)-((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-phenoxy-phosphorylamino)-propionate **(1.1.2(7));**
*iso-*propyl (S)-2-[((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-*p*-tolyl-phosphorylamino)-propionate **(1.1.2(8));**
*iso-*propyl (S)-2-((S)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(9));**
*iso-*propyl (S)-2-((R)-{(2R,3R,5R)-5-[4-(2- dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(10));**
*iso-*propyl (S)-2-[(S)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(11));**
*iso-*propyl (S)-2-[(R)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(12));**
methyl (S)-2-{(((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(13));**
*iso-*propyl (S)-2-[((2R,3R,5R)-4,4-difluoro-3-hydroxy-5-{4-[((S)-1-methylpyrrolidine-3-carbonyl)-amino]-2-oxo-2H-pyrimidin-1-yl}-tetrahydro-furan-2-ylmethoxy)-phenoxy-phosphorylamino)-propionate **(1.1.2(14));**
*iso-*propyl (S)-2-[((2R,3R,5R)-4,4-difluoro-3-hydroxy-5-{4-[((S)-1-methylpyrrolidine-4-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(1));**
*iso-*propyl (R)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(2));**
*iso-*propyl (R)-2-[((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(3));**
*iso-*propyl (S)-2-((S)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(4));**
*iso-*propyl (S)-2-((R)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(5));**
*iso-*propyl (S)-2-[(S)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(6));**
*iso-*propyl (S)-2-[(R)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(7)).**

The subject of the present invention is a pharmaceutical composition for prophylaxis and treatment of viral infections including hepatitis C in the form of tablets, capsules or injections, placed in pharmaceutically acceptable packaging, not necessarily containing inhibitor of inosine-5-monophosphate dehydrogenase and/or inhibitor of hepatitis C protease NS3 and/or inhibitor of hepatitis C protease NS3/4A, and/or inhibitor of RNA-polymerase NS5A, and comprising a therapeutically effective amount of a compound of the general formulas **1, 2** or **3,** or stereisomers, salts, hydrates, solvates or crystalline forms thereof.

The more preferable is a pharmaceutical composition comprising a therapeutically effective amount of substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formula **1.1** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formula **1.2.**

The more preferable is also a pharmaceutical composition comprising a therapeutically effective amount of substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formulas **1.1.1, 1.1.2** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formulas **1.2.1, 1.2.2.**

The most preferable is a pharmaceutical composition comprising a therapeutically effective amount of compound selected from:
(*S*)-*iso*-propyl 2-{[(2*R*,3*S*,5*R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1));**
(2R,3S,5R)-5-(4-amino-2-oxo-2H-pyrimidin-1-yl)-4,4-difluoro-2-[((S)-1-*iso*propoxycarbonyl-ethylamino)-phenoxy-phosphorylaminomethyl]-tetrahydro-furan-3-yl (S)-1-methyl-pyrrolidine-2-carboxylate **(1.1.1(2));**
(2R,3R)-5-(4-amino-2-oxo-2H-pyrimidin-1-yl)-4,4-difluoro-2-[(S)-((S)-1-*iso*propoxycarbonyl-ethylamino)-phenoxy-phosphorylaminomethyl]-tetrahydro-furan-3-yl (S)-2-*tert*-butoxycarbonylamino-3-methyl-butanoate **(1.1.1(3));**
(2R,3R)-5-(4-amino-2-oxo-2H-pyrimidin-1-yl)-4,4-difluoro-2-[(R)-((S)-1-isopropoxycarbonyl-ethylamino)-phenoxy-phosphorylaminomethyl]-tetrahydro-furan-3-yl (S)-2-*tert*-butoxycarbonylamino-3-methylbutanoate **(1.1.1(4));**
*iso-*propyl (S)-2-({(2R,3R,5R)-5-[4-(2-dimethylamino-acetylamino)-2-oxo-2H-pyrimidin-1-yl]- 4,4-difluoro-3-hydroxy-tetrahydro-furan-2-ylmethoxy]-phenoxy-phosphorylamino}-propionate **(1.1.2(1));**
*iso-*propyl (S)-2-({(2R,3R,5R)-5-[4-((R)-2-*tert*-butoxycarbonylamino-3-methyl-butyrylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(2));**
*iso-*propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy]-phenoxy-phosphorylamino}-propionate **(1.1.2(3));**
methyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)]-phenoxy-phosphorylamino}-propionate **(1.1.2(4));**
*iso-*propyl (R)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-[(4-chlorophenoxy)-((2R,3R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(6));**
*iso-*propyl (S)-2-[(2,4-dichlorophenoxy)-((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-phenoxy-phosphorylamino)-propionate **(1.1.2(7));**
*iso-*propyl (S)-2-[((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-*p*-tolyl-phosphorylamino)-propionate **(1.1.2(8));**
*iso-*propyl (S)-2-((S)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(9));**
*iso-*propyl (S)-2-((R)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(10));**
*iso-*propyl (S)-2-[(S)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(11));**
*iso-*propyl (S)-2-[(R)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(12));**
methyl (S)-2-{[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(13));**
*iso-*propyl (S)-2-[((2R,3R,5R)-4,4-difluoro-3-hydroxy-5-{4-[((S)-1-methylpyrrolidine-3-carbonyl)-amino]-2-oxo-2H-pyrimidin-1-yl}-tetrahydro-furan-2-ylmethoxy)-phenoxy-phosphorylamino)-propionate **(1.1.2(14));**
*iso-*propyl (S)-2-[((2R,3R,5R)-4,4-difluoro-3-hydroxy-5-{4-[((S)-1-methylpyrrolidine-4-carbonyl)-amino]-2-oxo-2H-pyrimidin-1-yl}-tetrahydro-furan-2-ylmethoxy)-phenoxy-phosphorylamino)-propionate **(1.1.2(5));**
*iso-*propyl (S)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy- phosphorylamino)-propionate **(1.2.2(1));**
*iso-*propyl (R)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(2));**
*iso-*propyl (R)-2-[((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(3));**
*iso-*propyl (S)-2-((S)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(4));**
*iso-*propyl (S)-2-((R)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(5));**
*iso-*propyl (S)-2-[(S)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(6));**
*iso-*propyl (S)-2-[(R)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(7)).**

The most preferable is a pharmaceutical composition, in which as an inhibitor of inosine-5-monophosphate dehydrogenase is used Ribamidin, as an inhibitor of hepatitis C protease NS3 is used Asunaprevir (BMS-650032), as an inhibitor of hepatitis C protease NS3/4A is used Sofosbuvir (TMC435), and as an inhibitor of RNA polymerase NS5A is used Daclatasvir (BMS-790052) or Declatasvir (GS-5885).

The subject of the present invention is a medicament comprising as an active component an effective amount of a compound of the general formulas **1, 2** or **3,** stereoisomers, salts, hydrates, solvates or crystalline forms thereof for treatment of any condition caused by virus hepatitis C.

The more preferable is a medicament comprising as an active component an effective amount of substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formula **1.1** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formula **1.2.**

The more preferable is also a medicament comprising as an active component an effective amount of substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formulas **1.1.1, 1.1.2** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formulas **1.2.1, 1.2.2.**

The most preferable is a medicament, comprising as an active component an effective amount of a compound selected from:
(S)-*iso*-propyl 2-{[(*2R,3S,5R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1));**
(2R,3S,5R)-5-(4-amino-2-oxo-2H-pyrimidin-1-yl)-4,4-difluoro-2-[((S)-1-*iso*propoxycarbonyl-ethylamino)-phenoxy-phosphorylaminomethyl]-tetrahydro-furan-3-yl (S)-1-methyl-pyrrolidine-2-carboxylate **(1.1.1(2));**
(2R,3R)-5-(4-amino-2-oxo-2H-pyrimidin-1-yl)-4,4-difluoro-2-[(S)-((S)-1-*iso*propoxycarbonyl-ethylamino)-phenoxy-phosphorylaminomethyl]-tetrahydro-furan-3-yl (S)-2-*tert*-butoxycarbonylamino-3-methyl-butanoate **(1.1.1 (3));**
(2R,3R)-5-(4-amino-2-oxo-2H-pyrimidin-1-yl)-4,4-difluoro-2-[(R)-((S)-1-isopropoxycarbonyl-ethylamino)-phenoxy-phosphorylaminomethyl]-tetrahydro-furan-3-yl (S)-2-*tert*-butoxycarbonylamino-3-methyl-butanoate **(1.1.1 (4));**
*iso*-propyl (S)-2-({(2R,3R,5R)-5-[4-(2-dimethylamino-acetylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(1));**
*iso*-propyl (S)-2-({(2R,3R,5R)-5-[4-((R)-2-*tert*-butoxycarbonylamino-3-methyl-butyrylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(2));**
*iso*-propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy]-phenoxy-phosphorylamino}-propionate **(1.1.2(3));**
methyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)]-phenoxy-phosphorylamino}-propionate **(1.1.2(4));**
*iso*-propyl (R)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(5));**
*iso*-propyl (S)-2-[(4-chlorophenoxy)-((2R,3R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(6));**
*iso*-propyl (S)-2-[(2,4-dichlorophenoxy)-((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-phenoxy-phosphorylamino)-propionate **(1.1.2(7));**
*iso*-propyl (S)-2-[((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-p-tolyl-phosphorylamino)-propionate **(1.1.2(8));**
*iso*-propyl (S)-2-((S)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(9));**
*iso*-propyl (S)-2-((R)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(10));**
*iso*-propyl (S)-2-[(S)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(11));**
*iso*-propyl (S)-2-[(R)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(12));**
methyl (S)-2-{[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(13));**
*iso*-propyl (S)-2-[((2R,3R,5R)-4,4-difluoro-3-hydroxy-5-{4-[((S)-1-methylpyrrolidine-3-carbonyl)-amino]-2-oxo-2H-pyrimidin-1-yl}-tetrahydro-furan-2-ylmethoxy)-phenoxy-phosphorylamino)-propionate **(1.1.2(14));**
*iso*-propyl (S)-2-[((2R,3R,5R)-4,4-difluoro-3-hydroxy-5-{4-[((S)-1-methylpyrrolidine-4-carbonyl)-amino]-2-oxo-2H-pyrimidin-1-yl}-tetrahydro-furan-2-ylmethoxy)-phenoxy-phosphorylamino)-propionate **(1.1.2(5));**
*iso*-propyl (S)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(1));**
*iso*-propyl (R)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(2));**
*iso*-propyl (R)-2-[((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(3));**
*iso*-propyl (S)-2-((S)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(4));**
*iso*-propyl (S)-2-((R)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(5));**
*iso*-propyl (S)-2-[(S)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(6));**
*iso*-propyl (S)-2-[(R)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(7)).**

Medicaments could be administered peroral or parenterally (for example, intravenous, subcutaneous, intraperitoneally, local or rectal). Clinical dose of active component, pharmaceutical composition or combination product, including pharmaceutically effective amount of active component at patients can be corrected depending on: therapeutic efficiency and bio-accessibility of active ingredients in patients' organism, rate of their exchange and removal from organism, age, gender, and severity of patient's symptoms. Thus, the daily intake for adults normally is 10∼500 mg, preferably 50∼300 mg. While preparing pharmaceutical compositions as a dose unit the above effective dose is to be taken into consideration, at this, each dose unit of composition contains 10∼500 mg of active component, preferably - 50 ∼ 300 mg. Following the instructions of physician or pharmacist, the medicaments may be taken several times over specified periods of time (preferably, from one to six times).

The subject of the present invention is a method for treatment of a disease caused by hepatitis C virus, which includes introduction of therapeutically effective amount of a compound of the general formulas **1, 2** or **3,** or stereoisomers, salt, hydrate, solvate, crystalline form thereof, or pharmaceutical composition comprising compounds of the general formulas **1, 2** or **3,** or stereoisomers, salt, hydrate, solvate, crystalline form thereof.

The more preferable method for treatment of a disease caused by hepatitis C virus, in patients in need thereof is a method, which includes introduction of therapeutically effective amount of substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formula **1.1** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formula **1.2.**

The more preferable method for treatment of a disease caused by hepatitis C virus, in patients in need thereof is also a method, which includes introduction of therapeutically effective amount of substituted alkyl (S)-2-{[(2R,3S,5R)-5-(4-amino-2-oxo-2H-pyrimidin-1-yl)-4,4-difluoro-3-hydroxy-tetrahydro-furan-2-ylmethoxy]-phenoxy-phosphorylamino}-propionate of the general formulas **1.1.1, 1.1.2** or substituted alkyl (S)-2-{[(2R,3S,4R,5R)-5-(4-amino-2-oxo-2H-pyrimidin-1-yl)-4,4-difluoro-3-hydroxy-4-methyl-4-fluoro-tetrahydro-furan-2-ylmethoxy]-phenoxy-phosphorylamino}-propionate of the general formulas **1.2.1, 1.2.2.**

The most preferable method for treatment of a disease caused by hepatitis C virus, in patients in need thereof is a method, which includes introduction of therapeutically effective amount of a compound selected from:
(S)-*iso*-propyl 2-{[(*2R,3S,5R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1));**
(2R,3S,5R)-5-(4-amino-2-oxo-2H-pyrimidin-1-yl)-4,4-difluoro-2-[((S)-1-*iso*propoxycarbonyl-ethylamino)-phenoxy-phosphorylaminomethyl]-tetrahydro-furan-3-yl (S)-1-methyl-pyrrolidine-2-carboxylate **(1.1.1(2));**
(2R,3R)-5-(4-amino-2-oxo-2H-pyrimidin-1-yl)-4,4-difluoro-2-[(S)-((S)-1-*iso*propoxycarbonyl-ethylamino)-phenoxy-phosphorylaminomethyl]-tetrahydro-furan-3-yl (S)-2-*tert*-butoxycarbonylamino-3-methyl-butanoate **(1.1.1 (3));**
(2R,3R)-5-(4-amino-2-oxo-2H-pyrimidin-1-yl)-4,4-difluoro-2-[(R)-((S)-1-isopropoxycarbonyl-ethylamino)-phenoxy-phosphorylaminomethyl]-tetrahydro-furan-3-yl (S)-2-*tert*-butoxycarbonylamino-3-methylbutanoate **(1.1.1 (4));**
*iso*-propyl (S)-2-({(2R,3R,5R)-5-[4-(2-dimethylamino-acetylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(1));**
*iso*-propyl (S)-2-({(2R,3R,5R)-5-[4-((R)-2-*tert*-butoxycarbonylamino-3-methyl-butyrylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(2));**
*iso*-propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy]-phenoxy-phosphorylamino}-propionate **(1.1.2(3));**
methyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino}-propionate **(1.1.2(4));**
*iso*-propyl (R)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(5));**
*iso*-propyl (S)-2-[(4-chlorophenoxy)-((2R,3R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(6));**
*iso*-propyl (S)-2-[(2,4-dichlorophenoxy)-((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-phenoxy-phosphorylamino)-propionate **(1.1.2(7));**
*iso*-propyl (S)-2-[((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-p-tolyl-phosphorylamino)-propionate **(1.1.2(8));**
iso-propyl (S)-2-((S)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(9));**
*iso*-propyl (S)-2-((R)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(10));**
*iso*-propyl (S)-2-[(S)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(11));**
*iso*-propyl (S)-2-[(R)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(12));**
methyl (S)-2-{[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(13));**
*iso*-propyl (S)-2-[((2R,3R,5R)-4,4-difluoro-3-hydroxy-5-{4-[((S)-1-methylpyrrolidine-3-carbonyl)-amino]-2-oxo-2H-pyrimidin-1-yl}-tetrahydro-furan-2-ylmethoxy)-phenoxy-phosphorylamino)-propionate **(1.1.2(14));**
*iso*-propyl (S)-2-[((2R,3R,5R)-4,4-difluoro-3-hydroxy-5-{4-[((S)-1-methylpyrrolidine-4-carbonyl)-amino]-2-oxo-2H-pyrimidin-1-yl}-tetrahydro-furan-2-ylmethoxy)-phenoxy-phosphorylamino)-propionate **(1.1.2(5));**
*iso*-propyl (S)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy- phosphorylamino)-propionate **(1.2.2(1));**
*iso*-propyl (R)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(2));**
*iso*-propyl (R)-2-[((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(3));**
*iso*-propyl (S)-2-((S)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(4));**
*iso*-propyl (S)-2-((R)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(5));**
*iso*-propyl (S)-2-[(S)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(6));**
*iso*-propyl (S)-2-[(R)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(7)).**

Clinical dose of pharmaceutical composition for prophylaxis and treatment of viral infections including hepatitis C in the form of tablets, capsules or injections placed in pharmaceutically acceptable pakage, not nessaserily comprising inhibitor of inosine-5-monophosphate dehydrogenase and/or inhibitor of hepatitis C NS3 protease, and/or inhibitor of hepatitis C NS3/4A protease, and/or inhibitor of RNA-polymerase NS5A, comprising a compound of the general formulas **1, 2** or **3,** or stereoisomers, salts, hydrates, solvates, or crystalline forms thereof at patients may be corrected depending on: therapeutic efficiency and bio-accessibility of active ingredients in patients' organism, rate of their exchange and removal from organism, age, gender, and severity of patient's symptoms. Thus, the daily intake for adults normally is 10∼500 mg. Thus, according to the present invention, while preparing pharmaceutical compositions as a dose unit the above effective dose is to be taken into consideration, at this, each dose unit of composition should contain 10∼500 mg of novel nucleoside inhibitor of HCV RNA-polymerase NS5B of the general formula **1, 2** or **3,** or stereoisomer, pharmaceutically acceptable salt, hydrate, solvate, crystalline or polycryctalline form thereof. Following the instructions of physician or pharmacist, the medicaments may be taken several times over specified periods of time (preferably, from one to six times).

For combination therapy any classes of agents that may be useful when combined with the compounds of the present invention to form pharmaceutical composition, and which may mean, for example, nucleoside and non-nucleoside inhibitors of the HCV polymerase, protease inhibitors, helicase inhibitors, and medicinal agents that functionally inhibit the internal ribosomal entry site (IRES) and other medicaments that inhibit HCV cell attachment or virus entry into cells, HCV RNA translation, replication or HCV maturation or virus release. Specific compounds in these classes and useful in this invention include, but are not limited to, macrocyclic, heterocyclic and linear HCV protease inhibitors such as telaprevir (VX-950), boceprevir (SCH-503034), narlaprevir (SCH-900518), ITMN-191 (R-7227), TMC-435350 (a.k.a. TMC-435), MK-7009, BI-201335, BI-2061 (ciluprevir), ACH-1625, ACH-1095 (HCV NS4A protease co-factor inhibitor), VX-500, VX-813, PHX-1766, PHX2054, IDX-136, IDX-316, ABT-450 EP-013420 (and congeners) and VBY-376; the Nucleosidic HCV polymerase (replicase) inhibitors useful in the invention include, but are not limited to, R7128, IDX-184, IDX-102, R1479, UNX-08189, PSI-6130, PSI-938 and PSI-879 and various other nucleoside and nucleotide analogs and HCV inhibitors including (but not limited to) those derived as 2'-C-methyl modified nucleoside and nucleotide; and 7'-deaza modified nucleoside and nucleotide. Non-nuclosidic HCV polymerase (replicase) inhibitors useful in the invention, include, but are not limited to, HCV-796, HCV-371, VCH-759, VCH-916, VCH-222, ANA-598, MK-3281, ABT-333, ABT-072, PF-00868554, BI-207127, GS-9190, A-837093, JKT-109, GL-59728 and GL-60667.

Besids, the novel nycleoside inhibitors of HCV RNA-polymerase NS5B of the general formulas **1, 2** or **3** could be used in pharmaceutical composition in combination with cyclophyllin and immunophyllin antagonists (for example, without limitation, DEBIO compounds, NM-811, as well as cyclosporine and its derivatives), kinase inhibitors, inhibitors of heat shock proteins (for example, HSP90, HSP70), other immunomodulatory agents that may include, without limitation, interferons (alpha-, beta-, omega-, gamma-, lambda or synthetic), Intron A™, Roferon- A™, Canferon-A300™, Advaferon™, Infergen™, Humoferon™, Sumiferon MP™, Alfaferon™, IFN-β™, Feron™, and the like, polyethylene glycol derivatized (pegylated) interferon compounds, such as: PEG interferon-α-2a (Pegasys™), PEG interferon-α-2b (PEGIntron™), pegylated IFN-α-con 1 and the like; long acting formulations and derivatives of interferon compounds, such as albumin-fused interferon, Albuferon™, Locteron™, and the like; interferons with various types of controlled delivery systems (e.g. ITCA-638, omega-interferon delivered by the DUROS subcutaneous delivery system); compounds that stimulate the synthesis of interferon in cells, such as resiquimod and the like; interleukins; compounds that enhance the development of type 1 helper T cell response, such as SCV-07 and the like; TOLL-like receptor agonists, such as: CpG-10101 (action), isotorabine, ANA773 and the like; thymosin α-1, ANA-245 and ANA-246, histamine dihydrochloride, propagermanium; tetrachlorodecaoxide; ampligen; IMP-321; KRN-7000; antibodies, such as: civacir, XTL-6865 and the like and prophylactic and therapeutic vaccines, such as: Inno Vac, HCV E1 E2/MF59 and the like. In addition, any of the above-described methods involving administration of an NS5B inhibitor, a Type 1 interferon receptor agonist (e.g., an IFN-α) and a Type Π interferon receptor agonist (e.g., IFN-γ) can be augmented by administration of an effective amount of TNF-α antagonist. Exemplary, non-limiting TNF-α antagonists that are suitable for use in such combination therapies include ENBREL™ and HUMIRA™.

In addition, the novel nucleoside inhibitor of HCV RNA polymerase NS5B of the general formulas **1, 2** or **3** may be used in pharmaceutical composition in combination with antiprotozoans and other antivirals thought to be effective in the treatment of HCV infection, such as, the prodrug nitazoxanide. Nitazoxanide can be used as an agent in combination with the compounds disclosed in this invention as well as in combination with other agents useful in treating HCV infection such as peginterferon alfa-2a and ribavarin (see, for example, Rossignol, JF and Keeffe, EB, Future Microbiol. 3:539-545, 2008).

The novel nucleoside inhibitor of HCV RNA polymerase NS5B of the general formulas **1, 2** or **3** may be used in pharmaceutical composition in combination with alternative forms of interferons and pegylated interferons, ribavirin or its analogs (e.g., Tarabavarin, levovirion), micro RNA, small interfering RNA compounds (e.g., SIRPLEX-140-N and the like), nucleotide or nucleoside analogs, immonoglobulins, hepatoprotectants, anti-inflammatory agents and other inhibitiors of NS5B. Inhibitors of other targets in the HCV life cycle include NS3 helicase inhibitors; NS4A co-factor inhibitors, antisense oligonucleotide inhibitors, such as ISIS-14803, AVI-4065 and the like; vector-encoded short hairpin RNA (shRNA); HCV specific ribozymes such as heptazyme, RPI, 139199 and the like; entry inhibitors such as HepeX-C, HuMax-HepC and the like; alpha glucosidase inhibitors such as celgosivir, UT-231B and the like; KPE-02003002 and BIVN 401 and IMPDH inhibitors. Other illustrative compounds - HCV inhibitor compounds include those disclosed in the known scientific and patent publications.

Additionally, combinations of, for example, ribavirin and interferon may be administered as combination therapy with, at least one novel nucleoside inhibitor of HCV RNA polymerase NS5B of the general formulas **1, 2** or **3** or stereoisomer, pharmaceutically acceptable salt, hydrate, solvate, crystalline or polycrystalline form thereof. The present invention is not limited to the aforementioned classes or compounds and contemplates known and new compounds and combinations of biologically active agents. It is intended that combination therapies of the present invention include any chemically compatible combination of novel nucleoside inhibitor of HCV RNA polymerase NS5B of the general formulas **1, 2** or **3** of this inventive group with other compounds of the inventive group or other compounds outside of the inventive group, as long as the combination does not eliminate the anti-viral activity of the compound of this inventive group or the anti-viral activity of the pharmaceutical composition itself.

Combination therapy can be sequential, that is treatment with one agent first and then a second agent (for example, where each treatment comprises a different compound of the present invention or where one treatment comprises a compound of the present invention and the other comprises one or more biologically active agents) or it can be treatment with both agents at the same time. Sequential therapy can include a reasonable time after the completion of the first therapy before beginning the second therapy. Treatment with both agents at the same time can be in the same daily dose or in separate doses. Combination therapy need not be limited to two agents and may include three or more agents. The dosages for both concurrent and sequential combination therapy will depend on absorption, distribution, metabolism and excretion rates of the components of the combination therapy as well as other factors known to one of skill in the art. Dosage values will also vary with the severity of the condition to be alleviated. For any particular subject specific dosage regimens and schedules may be adjusted over time according to the individual's need and the professional judgement of the person administering or superivising the administration of the combination therapy. Although the foregoing invention has been described in some detail by way of illustration and example for purposes of clarity of understanding, it will be readily apparent to one of ordinary skill in the art in light of the teaching of this invention that certain changes and modifications may be made thereto without departing from the spirit and scope of the invention as defined in the appended claims.

### Best Embodiment of the invention

Below the invention is described by means of specific examples which illustrate, but not limit the scope of the invention.

### Examle 1.

### The general method for preparation of (S)-iso-propyl 2-{[(2R,3S,5R)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate (1.1.1(1)) and (2R,3S,5R)-5-(4-amino-2-oxo-2H-pyrimidin-1-yl)-4,4-difluoro-2-[((S)-1-iso-propoxycarbonyl-ethylamino)-phenoxy-phosphoryloxymethyl]-tetrahydro-furan-3-yl (S)-1-methyl-pyrrolidine-2-carboxylate (1.1.1(2)).

3.85 g (1.5 eq.) of Boc-anhydride was added to a solution of 2.24 g (11.2 mmol) of 4-amino-1-((2R,4R,5R)-3,3-difluoro-4-hydroxy-5-hydroxymethyl-tetrahydro-furan-2-yl)-1H-pyrimidine-2-one **(5(1))** in 8 ml of dimethylformamide. The reaction mixture was heated at 50 ° C for 18 h. In order to decompose the excess of Boc₂O 8 ml of water was added, the reaction mixture was cooled to room temperature, at stirring 16 ml of water was added. The suspension was filtered, the solid on filter was washed with water. The obtained *tert*-butyl [1-((2R,4R,5R)-3,3-difluoro-4-hydroxy-5-hydroxymethyl-tetrahydro-furan-2-yl)-2-oxo-1,2-dihydro-pyrimidin-4-yl]-carbamate **(5(2))** was dried *in vacuo.* Yield is 3.65 g (86%). HPLC of purity (UV254) was 98.3%. ¹H NMR (DMSO-D6, 300 MHz) δ 1.46 (s, 9H), 3.6-3.9 (m, 2H), 4.18 (m, 1H), 5,29 (br. s, 1H), 6.16 (t, J = 7.5 Hz, 1H), 6.30 (d, J = 6.6 Hz, 1H), 7.06 (d, J = 7.8, 1H), 8.18 (d, J = 7.8 Hz, 1H), 10.52 (s, 1H).

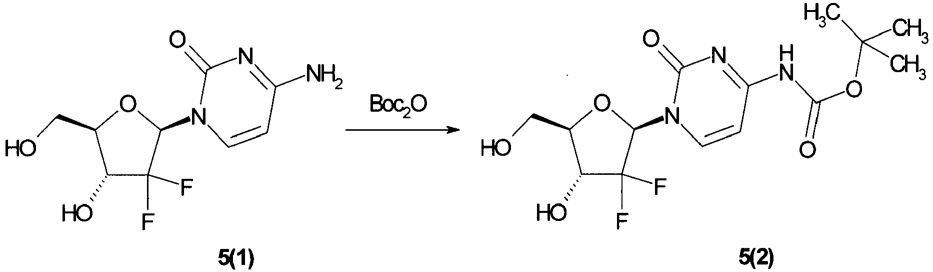

A solution of 1.8 mmol of prepared product **5(2)** and 10.98 mmol (875 mcl) of *N-*methylimidazole in 10 ml of THF was stirred for 30 min at 0 °C, then a solution of 5.49 mmol of (*S*)-*iso*-propyl-2-(chloro(phenoxy)phosphorylamino)propanoate **4(1)** in 10 ml of methylene chloride was dropped to it at 0 °C and the resultant mixture was stirred for 16 h at room temperature. 0.3 ml of Methanol was added, and the reaction mixture was stirred for 10 min. Then, 20 ml of ethyl acetate was added, the reaction mixture was washed with 5 % solution of HCl, saturated solution of NaHCO₃, dried over Na₂SO₄ and evaporated *in vacuo.* The residue was subjected to chromatography on silica, eluent - chloroform : methanol 9:1. It gave (S)-*iso-*propyl 2-{[(2R,3S,5R)-3-hydroxy-5-(4-*tert*-butoxycarbonylamino-2-oxo-2H-pyrimidin-1-yl)-tetrahydro-furan-2-ylmethoxy]-phenoxy-phosphorylamino}-propionate **(9(1))**, LC-MS m/e 633 (M+1).

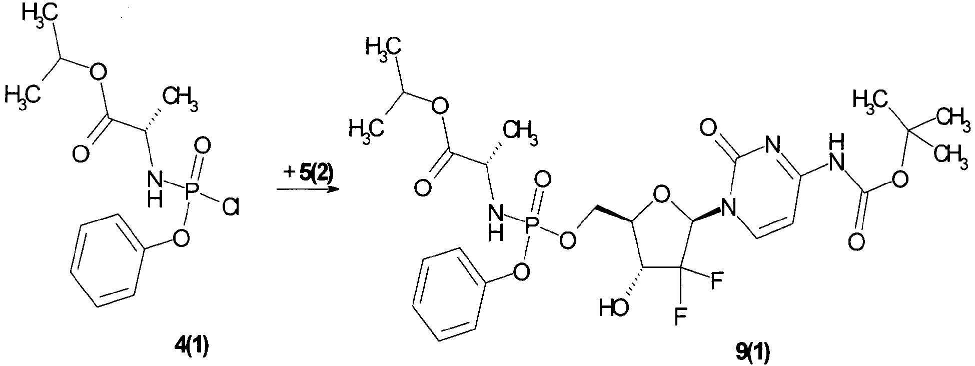

0.5 Mmol of 2-dimethylaminoacetic acid or (S)-1-methylpyrrolidine-2-carboxylic acid was added to a mixture of 0.2 mmol of the prepared compound **9(1)**, 115 mg (0.6 mmol) of *N*-(3-dimethylaminopropyl)-*N'*-ethylcarbodiimide (EDAC) hydrochloride and 14 mg (0.115 mmol) of 4-dimethylaminopyridine (DMAP) in 3 ml of acetonitrile, and the resultant mixture was stirred in argon atmosphere for 12 h. The mixture was diluted with 15 ml of chloroform and stirred for 3 h with saturated solution of NaHCO₃. Organic layer was dried over Na₂SO₄ and evaporated in vacuo. The residue was subjected to chromatography on silica, eluent - chloroform : methanol : triethylamine (80:1:2), it gave correspondingly (S)*-iso-*propyl 2-{[(2R,3R,5R)-5-(4-*tert*-butoxycarbonylamino-2-oxo-2H-pyrimidin-1-yl)-3-(2-dimethylamino-acetoxy)-4,4-difluoro-tetrahydro-furan-2-ylmethoxy]-phenoxy-phosphorylamino}-propionate **(10(1)),** LC-MS m/e 718 (M+1) or 2-{[(2R,3R,5R)-5-(4-*tert*-butoxycarbonylamino-2-oxo-2H-pyrimidin-1-yl)-4,4-difluoro-2-[((S)-1-iso-propoxycarbonyl-ethylamino)-phenoxy-phosphoryloxymethyl]-tetrahydro-furan-3-yl] (S)-1-methyl-pyrrolidine-2-carboxylate **10(2)** (R⁴ = (S)-1-methylpyrrolidine-2-carbonyl, LC-MS m/e 744 (M+1).

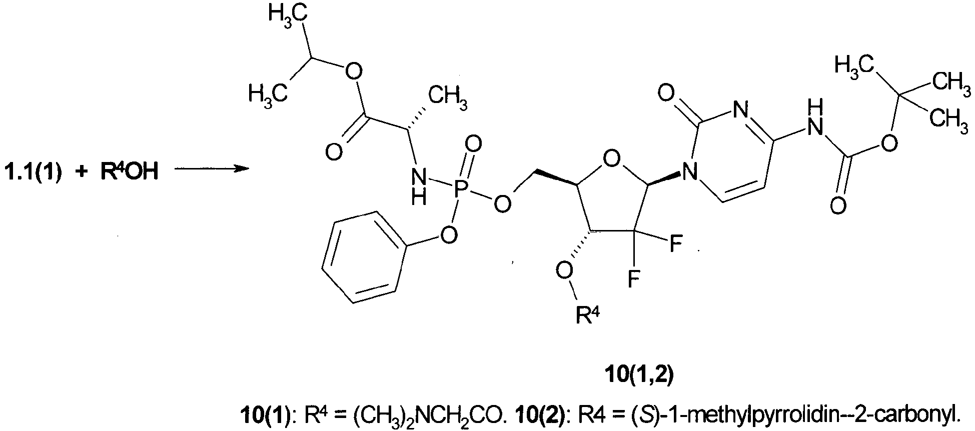

0.2 Mmol of prepared compound **10(1,2)** in 30% trifluoroacetic acid in methylene chloride at room temperature was stirred until the end of the reaction. LC-MS control. The solvent was evaporated *in vacuo,* the residue was purified by means of high-performance liquid chromatography. It gave correspondingly *iso*-propyl (*S*)-2-{[(*2R,3S,5R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1)),** LC-MS m/e 618 (M+1), or (2R,3S,5R)-5-(4-amino-2-oxo-2H-pyrimidin-1-yl)-4,4-difluoro-2-[((S)-1-*iso*-propoxycarbonyl-ethylamino)-phenoxy-phosphorylaminomethyl]-tetrahydro-furan-3-yl (S)-1-methyl-pyrrolidine-2-carboxylate **(1.1.1(2)),** LC-MS m/e 644 (M+1).

### Example 2.

### The general method for preparation of iso-propyl (S)-2-({(2R,3R,5R)-5-[4-((R)-2-tert-butoxycarbonylamino-3-methyl-butyrylamino)-2-oxo-2H-pyrimidin-1-yl]-4,4-difluoro-3-hydroxy-tetrahydro-furan-2-ylmethoxy}-phenoxy-phosphorylamino)-propionate (1.1.2(2)) and iso-propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate (1.1.2(3)).

230 Mg (1.37 mmol) of 1,1'-carbonyldiimidazole was added to a solution of 271 mg (1.25 mmol) of Boc-vaniline in 5 ml of methylene chloride and stirred for 30 min at room temperature. The prepared solution of imidazolide was dropped to a solution of 436 mg (0.82 mmol) of (S)-*iso*-propyl 2-{[(2R,3S,5R)-5-(4-amino-2-oxo-2H-pyrimidin-1-yl)-3-hydroxy-tetrahydro-furan-2-ylmethoxy]-phenoxy-phosphorylamino}-propionate **2** in 10 ml of methylene chloride. Then, the reaction mixture was refluxed for 16 h. The solution was evaporated *in vacuo,* the residue, LC-MS m/e 732 (M+1), was subjected to primary purification on silica, eluent ethyl acetate : hexane 2:1, chloroform : methanol 9:1. It gave two fractions, each of them was purified by means of high-performance liquid chromatography. It gave stereoisomers **1.1.2(2),** LC-MS m/e 732 (M+1) and **1.1.2(3),** LC-MS m/e 732 (M+1).

### Example 3.

### The general method for preparation of alkyl 2-((((2R,3R)-5-(4-aminooxopyrimidin-1(2H)-yl)-3-hydroxy-2--4,4-difluoro-tetrahydofuran-2-yl)methoxy)(phenoxy)phosphorylamino)-propanoates of the general formula 1-3.

A solution of 1.83 mmol of 2'-deoxy-2',2'-difluorocytidine and 10.98 mmol (875 mcl) of *N*-methylimidazole in 10 ml of THF was stirred for 30 min at 0 °C, then to it was added a solution of 5.49 mmol of (S)-alkyl-2-(chloro(phenoxy)phosphorylamino)propanoate in 10 ml of methylene chloride at 0 °C and the resultant mixture was stirred at room temperature for 16 h. To the reaction mixture 0.3 ml of methanol was added and srirred for 10 min. Then, 20 ml of ethyl acetate was added, the reaction mixture was washed with 5 % solution of HCl, saturated solution of NaHCO₃, dried over Na₂SO₄ and evaporated *in vacuo.* The residue was subjected to chromatography on silica, eluent chloroform : methanol 9:1. If needed, additional purification was carried out using HPLC without acid. It gave desired product, including: (S)-*iso*-propyl 2-({(*2R,3R,5R*)-5-[4-(2-dimethylamino-acetylamino)-2-oxo-2H-pyrimidin-1-yl]-4,4-difluoro-3-hydroxy-tetrahydro-furan-2-ylmethoxy}-phenoxy-phosphorylamino)-propionate **(1.1.2(1)),** LC-MS m/e 618 (M+1), ¹H NMR (DMSO-D6, 400 MHz) δ 10.51 (br. s, 1H), 8.02 (m, 1H), 7.37 (m, 2H), 7.27 (m, 1H), 7.20 (m, 3H), 6.52 (br. m, 1H), 6.22 (q, *J* = 8.0 Hz, 1H), 6.13 (m, 1H), 4.86 (m, 1H), 4.26 (m, 4H), 3.80 (m, 1H), 3.17 (s, 2H), 2.27 (s, 6H), 1.22 (t, *J* = 6.8 Hz, 3H), 1.15 (m, 6H); (S)-*iso*-propyl 2-[((*2R,3R,5R*)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(4)),** LC-MS m/e 644 (M+1), ¹H NMR (DMSO-D6, 400 MHz) δ 10.31 (br. s, 1H), 8.04 (d. d, *J₁* = 14.8 Hz, *J₂* = 7.6 Hz, 1H), 7.33 (m, 3H), 7.20 (m, 3H), 6.54 (br. m, 1H), 6.23 (q, *J* = 8.0 Hz, 1H), 6.13 (m, 1H), 4.86 (m, 1H), 4.26 (m, 4H), 3.80 (m, 1H), 3.08 (m, 3H), 2.90 (m, 1H), 2.35 (s, 3H), 1.76 (m, 3H), 1.23 (t, *J* = 7.0 Hz, 3H), 1.15 (m, 6H); (S)-*iso*-propyl 2-((((2R,3R)-5-(4-aminooxopyrimidin-1(2*H*)-yl)-3-hydroxy-2--4,4-difluoro-tetrahydrofuran-2-yl)methoxy)(phenoxy)phosphorylamino)propanoate **(2),** LC-MS m/e 533 (M+1), ¹H NMR (DMSO-D6, 400 MHz) δ: 7.70 (d, *J* = 9.6 Hz, 1H), 7.40 (m, 4H), 7.21 (m, 3H), 6.30 (m, 1H), 6.20 (m, 1H), 5.80 (d, *J* = 9.6 Hz, 1H), 5.20 (br. s, 1H), 5.01 (m, 1H), 4.86 (m, 1H), 4.05 (m, 1H), 3.78 (m, 2H), 3.61 (m, 1H), 1.25 (m, 3H), 1.16 (m, 6H); (S)-*iso*-propyl 2-((((2*R*,3*R*)-3-acetoxy-5-(4-amino-2-oxopyrimidin-1(2*H*)-yl)-4,4-difluoro-tetrahydofuran-2-yl)methoxy)(phenoxy)phosphorylamino)propanoate **(3),** LC-MS m/e 575 (M+1), ¹H NMR (DMSO-D6, 400 MHz) δ: 7.49 (m, 3H), 7.36 (m, 2H), 7.18 (m, 3H), 6.26 (m, 1H), 6.08 (m, 1H), 5.77 (m, 1H), 5.37 (br. m, 1H), 4.86 (m, 1H), 4.35 (m, 3H), 3.79 (m, 1H), 2.15 (s, 3H), 1.22 (t, *J* = 6.8 Hz, 3H), 1.15 (m, 6H).

### Example 4.

### The general method for preparation of (S)-iso-propyl 2-({(2R,3R,5R)-5-[4-(2-dimethylamino-acetylamino)-2-oxo-2H-pyrimidin-1-yl]-4,4-difluoro-3-hydroxy-tetrahydro-furan-2-ylmethoxy}-phenoxy-phosphorylamino)-propionate (1.1.2(1)) and (S)-iso-propyl 2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-ptopionate (1.1.2(4)).

To a mixture of 0.2 mmol of compound **6,** 115 mg (0.6 mmol) of N-(3-dimethylaminopropyl)-*N'*-ethylcarbodiimide (EDAC) hydrochloride and 14 mg (0.115 mmol) of 4-dimethylaminopyridine (DMAP) in 3 ml of acetonitrile was added 0.5 mmol of corresponding amino acid **8** and the resultant mixture was stirred in argon atmosphere for 12 h. The mixture was diluted with 15 ml of chloroform and stirred for 3 h with saturated solution of NaHCO₃. Organic layer was dried over Na₂SO₄ and evaporated *in vacuo.* The residue was subjected to chromatogtaphy on silica, eluent chloroform : methanol : triethylamine (80:1:2), it gave a desired product **1** (R⁶ = H), including: (S)-*iso*-propyl 2-({(*2R,3R,5R*)-5-[4-(2-dimethylamino-acetylamino)-2-oxo-2H-pyrimidin-1-yl]-4,4-difluoro-3-hydroxy-tetrahydro-furan-2-ylmethoxy}-phenoxy-phosphorylamino)-propionate **(1.1.2(1)),** LC-MS m/e 618 (M+1), ¹H NMR (DMSO-D6, 400 MHz) δ: 10.51 (br. s, 1H), 8.02 (m, 1H), 7.37 (m, 2H), 7.27 (m, 1H), 7.20 (m, 3H), 6.52 (br. m, 1H), 6.22 (q, *J* = 8.0 Hz, 1H), 6.13 (m, 1H), 4.86 (m, 1H), 4.26 (m, 4H), 3.80 (m, 1H), 3.17 (s, 2H), 2.27 (s, 6H), 1.22 (t, *J* = 6.8 Hz, 3H), 1.15 (m, 6H); (S)-*iso*-propyl 2-[((*2R,3R,5R*)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(4)),** LC-MS m/e 644 (M+1), ¹H NMR (DMSO-D6, 400 MHz) δ: 10.31 (br. s, 1H), 8.04 (d. d, *J₁* = 14.8 Hz, *J₂* = 7.6 Hz, 1H), 7.33 (m, 3H), 7.20 (m, 3H), 6.54 (br. m, 1H), 6.23 (q, *J* = 8.0 Hz, 1H), 6.13 (m, 1H), 4.86 (m, 1H), 4.26 (m, 4H), 3.80 (m, 1H), 3.08 (m, 3H), 2.90 (m, 1H), 2.35 (s, 3H), 1.76 (m, 3H), 1.23 (t, *J* = 7.0 Hz, 3H), 1.15 (m, 6H)

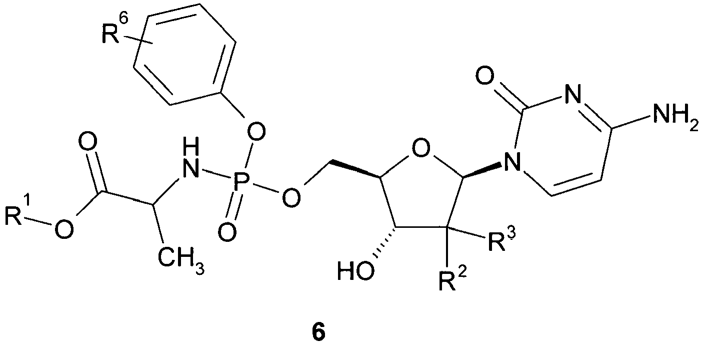

R⁵-OH 8

where R⁴ represents hydrogen, R⁵ represents optionally substituted α-aminoacyl, R¹, R², R³ and R⁶ have the above meanings.

### Example 5.

### (S)-iso-Propyl 2-((((2R,3R)-3-acetoxy-5-(4-amino-2-oxo-pyrimidin-1(2H)-yl)-4,4-difluoro-tetrahydrofuranyl)methoxy)(phenoxy)phosphorylamino) propanoate of the general formula 3.

To a mixture of 80 mg (0.15 mmol) (S)-*iso*-propyl 2-((((2*R*,3*R*)-3-hydroxy-5-(4-amino-2-oxopyrimidin-1(2*H*)-yl)-4,4-difluoro-tetrahydrofuryl)methoxy)(phenoxy)phosphorylamino) propanoate of the general formula **2,** 1.4 mg (0.01 mmol) of 4-dimethylaminopyridine (DMAP) and 34.6 mcl (0.25 mmol) of triethylamine in 2 ml of acetonitrile was added 21.3 mcl (0.23 mmol) acetic anhydride, and the resultant mixture was stirred in argon atmosphere for 12 h. Then, 40 mcl of methanol was added, the mixture was stirred for 3 h and evaporated in vacuo. The desired product was separated by means of HPLC without adding to eluent (gradient water-acetonitrile). Yield of (*S*)-*iso*-propyl 2-((((2*R*,3*R*)-3-acetoxy-5-(4-amino-2-oxopyrimidin-1(2*H*)-yl)-4,4-difluoro-tetrahydrofuran-2-yl)methoxy)(phenoxy) phosphorylamino)propanoate of the general formula **3** is 45 mg. LC-MS m/e 575 (M+1), ¹H NMR (DMSO-D6, 400 MHz) δ: 7.49 (m, 3H), 7.36 (m, 2H), 7.18 (m, 3H), 6.26 (m, 1H), 6.08 (m, 1H), 5.77 (m, 1H), 5.37 (br. m, 1H), 4.86 (m, 1H), 4.35 (m, 3H), 3.79 (m, 1H), 2.15 (s, 3H), 1.22 (t, *J* = 6.8 Hz, 3H), 1.15 (m, 6H).

### Example 6.

A pharmaceutical composition in the form of tablets. Starch (1600 mg), grained lactose (1600 mg), talcum (400 mg) and (*S*)-*iso*-propyl 2-((((2*R*,3*R*)-5-(4-aminooxopyrimidin-1(2*H*)-yl)-3-hydroxy-2--4,4-difluoro-tetrahydrofuran-2-yl)methoxy) (phenoxy)phosphorylamino)propanoate (1000 mg) were carefully mixed together and pressed into a brick. The prepared brick was crushed to granules and riddled through sieves, gathering granules of 14-16 mesh size. The obtained granules were pelletized into tablets of suitable form by 560 mg each.

### Example 7.

A pharmaceutical composition in the form of capsules. (*S*)-*iso*-propyl 2-((((2*R*,3*R*)-5-(4-aminooxopyrimidin-1(2*H*)-yl)-3-hydroxy-2--4,4-difluoro-tetrahydrofuran-2-yl)methoxy)(phenoxy)phosphorylamino)propanoate was carefully mixed with lactose powder in ratio 2:1. The resultant powdery mixture was packed by 300 mg into gelatinous capsules of suitable size.

### Example 8.

A pharmaceutical composition in the forn of injectable compositions for intramuscular, intraperitoneal or subcutaneous injections. (*S*)-*iso*-propyl 2-((((2*R*,3*R*)-5-(4-aminooxopyrimidin-1(2*H*)-yl)-3-hydroxy-2--4,4-difluorotetrahydrofuran-2-yl)methoxy) (phenoxy)phosphorylamino)propanoate (500 mg) was mixed with chlorobutanol (300 mg), propylene glycol (2 ml) and injectable water (100 ml). The resultant solution was filtered and placed into 1 ml ampoules which were sealed afterwards.

### Example 9.

A pharmaceutical composition in the form of tablets. Starch (1600 mg), grained lactose (1600 mg), talcum (400 mg) and (S)-*iso*-propyl 2-((((2*R*,3*R*)-5-(4-aminooxopyrimidin-1(2*H*)-yl)-3-hydroxy-2--4,4-difluoro-tetrahydrofuran-2-yl)methoxy)(phenoxy)phosphorylamino)propanoate (1000 mg) were carefully mixed together and pressed into a brick. The prepared brick was crushed to granules and riddled through sieves, gathering granules of 14-16 mesh size. The obtained granules were pelletized into tablets of suitable form by 560 mg each, which is used together with Ribamidin tablet.

### Example 10.

A pharmaceutical composition in the form of tablets. Starch (1600 mg), grained lactose (1600 mg), talcum (400 mg) and (*S*)-*iso*-propyl 2-((((2*R*,3*R*)-5-(4-aminooxopyrimidin-1(2*H*)-yl)-3-hydroxy-2--4,4-difluoro-tetrahydrofuran-2-yl)methoxy)(phenoxy)phosphorylamino)propanoate (1000 mg) were carefully mixed together and pressed into a brick. The prepared brick was crushed to granules and riddled through sieves, gathering granules of 14-16 mesh size. The obtained granules were pelletized into tablets of suitable form by 560 mg each, which is used together with Asunaprevir (BMS-650032) tablet.

### Example 11.

A pharmaceutical composition in the form of tablets. Starch (1600 mg), grained lactose (1600 mg), talcum (400 mg) and (*S*)-*iso*-propyl 2-((((2*R*,3*R*)-5-(4-aminooxopyrimidin-1(2*H*)-yl)-3-hydroxy-2-4,4-difluoro-tetrahydrofuran-2-yl)methoxy) (phenoxy)phosphorylamino)propanoate (1000 mg) were carefully mixed together and pressed into a brick. The prepared brick was crushed to granules and riddled through sieves, gathering granules of 14-16 mesh size. The obtained granules were pelletized into tablets of suitable form by 560 mg each, which is used together with Sofosbuvir (TMC435) tablet.

### Example 12.

A pharmaceutical composition in the form of tablets. Starch (1600 mg), grained lactose (1600 mg), talcum (400 mg) and (S)-*iso*-propyl 2-((((2*R*,3*R*)-5-(4-aminooxopyrimidin-1(2*H*)-yl)-3-hydroxy-2--4,4-difluoro-tetrahydrofuran-2-yl)methoxy) (phenoxy)phosphorylamino)propanoate (1000 mg) were carefully mixed together and pressed into a brick. The prepared brick was crushed to granules and riddled through sieves, gathering granules of 14-16 mesh size. The obtained granules were pelletized into tablets of suitable form by 560 mg each, which is used together with Daclatasvir (BMS-790052) or Declatasvir (GS-5885) tablet.

### Example 13.

### Determination of antiviral activity (EC₅₀) of nucleoside inhibitors of HCV RNA-polymerase NS5B of the general formulas 1, 2 and 3.

Antiviral activity (EC₅₀) of nucleoside inhibitors of HCV RNA-polymerase NS5B of the general formula **1, 2** and **3** (further - test compounds) was determined in the human hepatoma cell line Huh7, comprising subgenomic RNA-replicon HCV (genotype 1 b, 1 a and 2a). For quantitative estimation of viral replication was used a version of immune test ELISA on viral cor-antigen in 96-well plate. Cytotoxicity of the compounds was estimated in parallel regime. As a reference substance was used **PSI-7977.** Cells Huh7 were seeded in 96-well plate (7.5x10³ cells to each well in 100 µl of culture medium). Solutions of the tested compounds in DMEM medium {DMEM 1X; Source: Cellgro; Catalogue: 10-013-CV} were prepared immediately before use. Eleven serial three fold dilutions with concentrations from 20 nM to 0.2 nM were prepared. In 4 hours after seeding, serial dilutions of the compounds were added to the cells (100 µl to each well). Final concentration of tested compounds was varied from 10 nM to 0.1 pM, and DMSO - 0.5%. If it was necessary, higher concentrations of the test compounds were investigated. Each dilution of the compound was tested on two identical wells. Then the cells were incubated for three days at 37°C/5% CO₂ and fixed by addition of acetone/methanol (1:1) mixture in amount of 250 µl/well. In 1 min the cells were washed 3 times with PBS (Phosphate Buffered Saline) solution. Then the cells were blocked by addition of 10% fetal calf serum in PBS solution in amount of 150 µl/well for 1 h at room temperature. After that the cells were incubated with mouse monoclonal antibodies to cor-antigen HCV, clon C7-50 (Source: Affinity BioReagents; Catalogue: MA1-080) (100 µl/well, working dilution - 1:500 in 10% fetal calf serum in PBS solution) for 2 h at 37°C. The cells were washed 6 times with PBS/0.05% Tween 20 solution, then, they were incubated for 1 h with goat anti-mouse immunoglobulin antibodies (conjugated with horseradish peroxidase, 100 µl/well, working dilution - 1:2500 in 10% fetal calf serum in PBS solution). The cells were washed 6 times with PBS/0.05% Tween 20 solution, once with PBS solution, after that substrate (1 tablet of o-phenylenediamine (oPD) + 12 ml citrate/phosphate buffer + 5 µl 30% H₂O₂) in amount of 100 µl/well was added. The plates were kept for 30 min in the dark at room temperature. The reaction was arrested by the adding 2N H₂SO₄ in amount of 100 µl/well, and optical density (at wavelength 490 nm) was measured by means of multiscan plate reader Victor3 V 1420 (Perkin Elmer). IC₅₀ values (concentration of test compound, lowering the level of virus RNA-replicon by 50%) for every tested compound were calculated using XLfit 4 program. The results are shown in **Table 1.**

### Example 14.

### Cytotoxicity determination of nucleoside inhibitors of HCV RNA-polymerase NS5B of the general formulas 1, 2 and 3.

Cytotoxicity of the test compounds (CC₅₀) was investigated in experiments on the human hepatoma cell line Huh7. Metabolic activity of cells was determined using ATPLite kit (Perkin Elmer, Boston, USA) in accordance with manufacturer instructions. Cytotoxic action was estimated by seeding the cells into black microplate with transparent bottom (96 wells, 10⁴ cells to each well). Three independent repeats were used for each test compound. The tested compounds were added in 18 h, after that the cells were incubated together with the compounds for 96 h. Each well was washed two times with phosphate buffered saline (0.2 ml/well) and then the cells were lysed by adding cell buffer (50 µl/well) (all mentioned reagents are included in ATPLite kit). The microplate was incubated for 5 min on a rotating platform at 600 r/min, after that 50 µl of substrate solution (a part of ATPLite kit) was added into each well. The microplate was incubated for additional 5 min on a rotating platform at 600 r/min, kept for 10 min in the dark; after that luminescence was measured using TopCount NXT instrument (Packard, Perkin Elmer). CC₅₀ Value corresponding to concentration of tested compound at which 50% of cells were ruined was used as quantitative characteristic for cytotoxicity estimation. Calculation of CC₅₀ value: for calculation of inhibition effectiveness (% Inh) the following equation was used: % Inh = [(L^{pos} - L^{ex})/ L^{pos} - L^{neg})] * 100%, where L^{pos} - positive control, luminescence in the wells with cells without compounds; L^{neg} - negative control, luminescence in the wells with medium without cells; L^{ex} - luminescence in wells with a compound of definite concentration.. Then, CC₅₀ values were calculated using XLfit 4 program. The results are shown in **Table 1.** The following **Table 1** shows data on inhibitory activity of some novel nucleoside inhibitors.

HCV RNA-polymerase NS5B of the general formula **1,** formulas **2** and **3** and of standard **PSI-7977** in relation to subgenomic RNA-replicon HCV (genotypes 1 b, 1 a and 2a), from which it follows that new polymerase inhibitors are 2-6 times more active than the standard. Cytotoxicity of the novel compounds exceeds 10 000 nM.

**Table 1.**

| Activity (EC₅₀) and cytotoxicity (CC₅₀) of nucleoside inhibitors of HCV RNA-polymerase NS5B of the general formulas **1,** formulas **2** and **3.** | | | | |
|---|---|---|---|---|
| **Nº inhibitor** | **HCV genotypes (10% FBS)** | | | |
| | **1b** | | **1a** | **2a** |
| | **EC₅₀** | **CC₅₀** | **EC₅₀** | **EC₅₀** |
| | **nM** | | | |
| **PSI-7977 (Standard)** | 91.8 | > 10 000 | 292.2 | 88.4 |
| **1.1.2(1)** | 35,0 | > 10 000 | 105,9 | 115,6 |
| **1.1.2(2)** | 60.6 | > 10 000 | 95.6 | 78.9 |
| **1.1.2(3)** | 60.9 | > 10 000 | 182.1 | 69.4 |
| **1.1.2(6)** | 14.4 | > 10 000 | 123.5 | 79.3 |
| **2** | 13.9 | > 10 000 | 113.6 | 57.2 |
| **3** | 15,7 | > 10 000 | 141,0 | 61,0 |

Test results for novel inhibitors of the general formulas **1, 2, 3** show their high activity and low cytotoxicity. And, unexpectedly, the disclosed compounds turned out to be more active nucleoside HCV polymerase inhibitors NS5B then the most advanced inhibitor PSI-7977.

### Industrial applicability

The invention could be used in medicine, veterinary, biochemistry.

## Claims

1. Compounds of the general formula 1, or stereoisomers, pharmaceutically acceptable salts, and/or hydrates, solvates or crystalline forms thereof 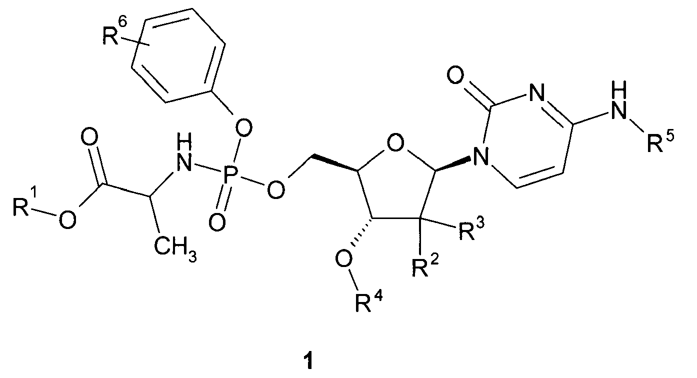 wherein
R¹ represents C₁-C₄ alkyl;
R² and R³ represents fluoro, or
R² represents fluoro, and R³ represents methyl;
R⁴ and R⁵ represents hydrogen, or
R⁴ represents C₁-C₆ acyl, and R⁵ represents hydrogen, or
R⁴ represents hydrogen, and R⁵ represents C₁-C₆ acyl, or
R⁴ represents hydrogen, and R⁵ represents optionally substituted α-aminoacyl, or
R⁴ represents optionally substituted α-aminoacyl, and R⁵ represents hydrogen, or
R⁶ represents hydrogen, methyl, methoxy or halogen.

2. Compounds according to claim 1, of the general formula **2** and of general formula **3**

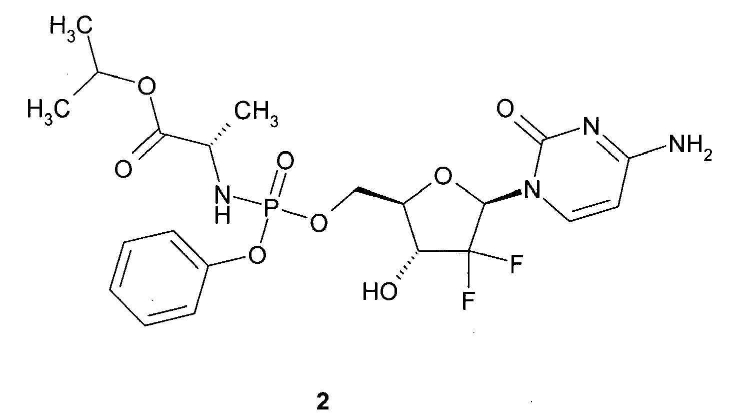

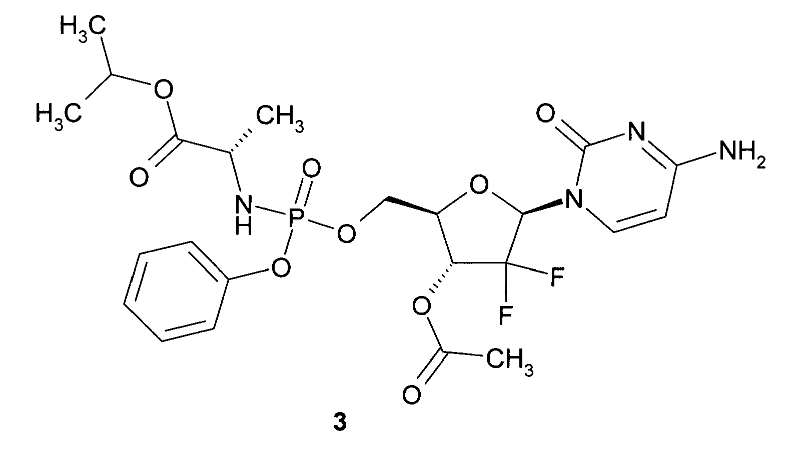

3. Compounds according to claim 1 of the general formula **1.1** and of the general formula **1.2,** 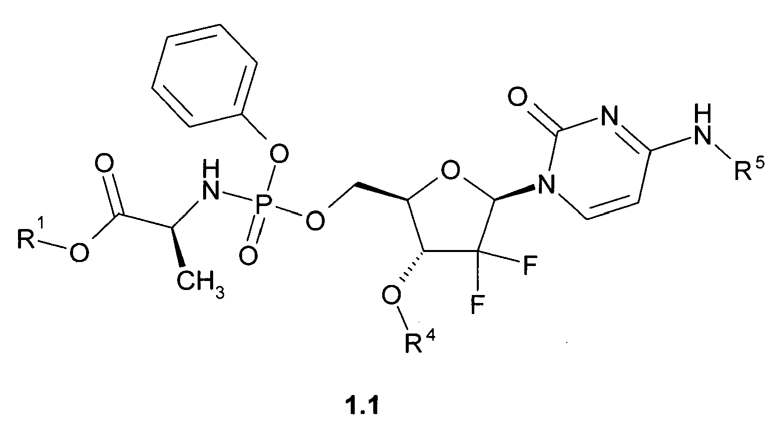 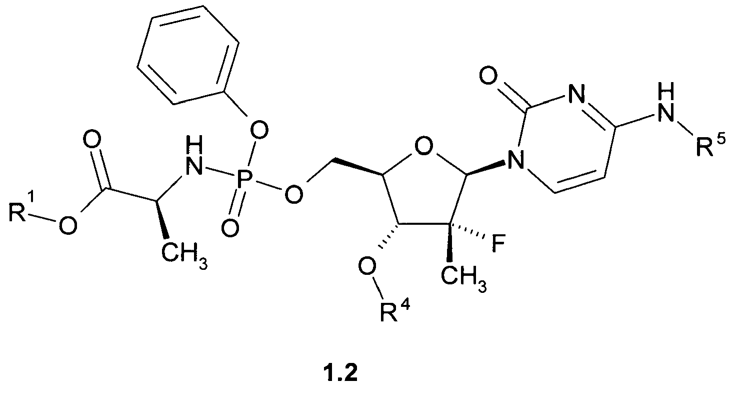 wherein
R¹, R⁴ and R⁵ have the above meanings.

4. Compounds according to claim 3 of the general formulas **1.1.1, 1.1.2** and of general formulas **1.2.1, 1.2.2** 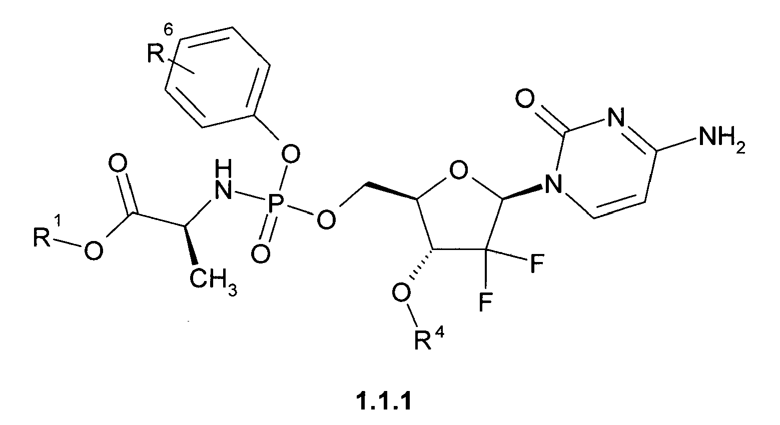 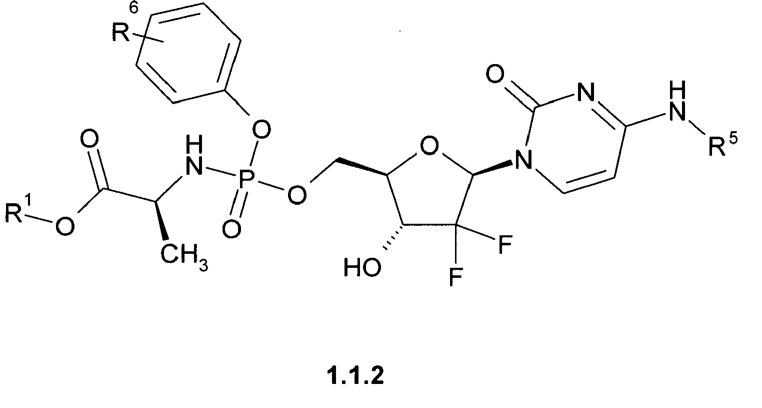 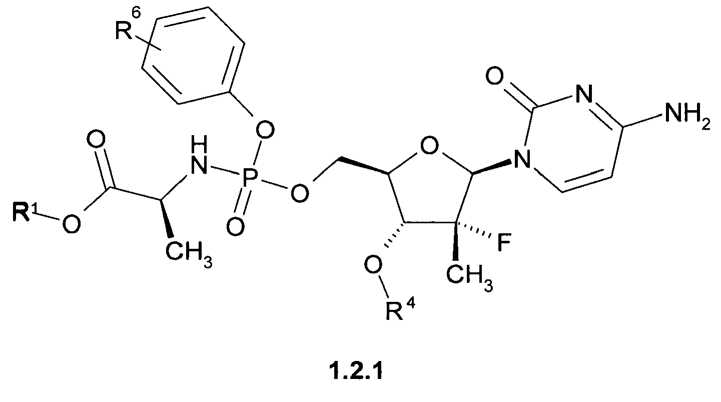 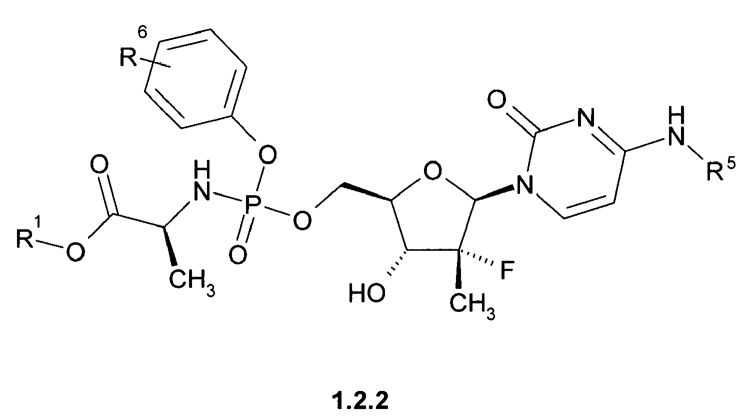 Wherein
R¹, R⁴, R⁵ and R⁶ have the above meanings.

5. Compounds according to claim 1, representing:
(*S*)-*iso*-propyl 2-{[(*2R*,*3S*,*5R*)-5-(4-amino-2-oxo-2H-pyrimidin-1-yl)-3-(2-dimethylamino-acetoxy)-4,4-difluoro-3-hydroxy-tetrahydro-furan-2-ylmethoxy]-phenoxy-phosphorylamino}-propionate **(1.1.1(1));**
(2R,3S,5R)-5-(4-amino-2-oxo-2H-pyrimidin-1-yl)-4,4-difluoro-2-[((S)-1-*iso*propoxycarbonyl-ethylamino)-phenoxy-phosphorylaminomethyl]-tetrahydro-furan-3-yl (S)-1-methyl-pyrrolidine-2-carboxylate **(1.1.1(2));**
(2R,3R)-5-(4-amino-2-oxo-2H-pyrimidin-1-yl)-4,4-difluoro-2-[(S)-((S)-1-*iso*propoxycarbonyl-ethylamino)-phenoxy-phosphorylaminomethyl]-tetrahydro-furan-3-yl (S)-2-*tert*-butoxycarbonylamino-3-methyl-butanoate **(1.1.1 (3));**
(2R,3R)-5-(4-amino-2-oxo-2H-pyrimidin-1-yl)-4,4-difluoro-2-[(R)-((S)-1-isopropoxycarbonyl-ethylamino)-phenoxy-phosphorylaminomethyl]-tetrahydro-furan-3-yl (S)-2-*tert*-butoxycarbonylamino-3-methylbutanoate **(1.1.1 (4));**
*iso*-propyl (S)-2-({(2R,3R,5R)-5-[4-(2-dimethylamino-acetylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(1));**
*iso*-propyl (S)-2-({(2R,3R,5R)-5-[4-((R)-2-*tert*-butoxycarbonylamino-3-methyl-butyrylamino)-2-oxo-2H-pyrimidin-1-yl]-4,4-difluoro-3-hydroxy-tetrahydro-furan-2-ylmethoxy]-phenoxy-phosphorylamino}-propionate **(1.1.2(2));**
*iso*-propyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy]-phenoxy-phosphorylamino}-propionate **(1.1.2(3));**
methyl (S)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)]-phenoxy-phosphorylamino}-propionate **(1.1.2(4));**
*iso*-propyl (R)-2-[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(5));**
*iso*-propyl (S)-2-[(4-chlorophenoxy)-((2R,3R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(6));**
*iso*-propyl (S)-2-[(2,4-dichlorophenoxy)-((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-phenoxy-phosphorylamino)-propionate **(1.1.2(7));**
*iso*-propyl (S)-2-[((2R,3R)-4,4-difluoro-3-hydroxy-5-{4-[((S)-1-methyl-pyrrolidine-2-carbonyl)-amino]-2-oxo-2H-pyrimidin-1-yl}-tetrahydro-furan-2-ylmethoxy)-*p*-tolyl-phosphorylamino)-propionate **(1.1.2(8));**
*iso*-propyl (S)-2-((S)-{(2R,3R,5R)-5-[4-(2-dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(9));**
*iso*-propyl (S)-2-((R)-{(2R,3R,5R)-5-[4-(2- dimethylaminoacetylamino)-2-oxo-2H-pyrimidin-1-yl]-4,4-difluoro-3-hydroxy-tetrahydro-furan-2-ylmethoxy)-phenoxy-phosphorylamino)-propionate **(1.1.2(10));**
*iso*-propyl (S)-2-[(S)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(11));**
*iso*-propyl (S)-2-[(R)-((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(12));**
methyl (S)-2-{[((2R,3R,5R)-4,4-difluoro-3-hydroxy-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.1.2(13));**
*iso*-propyl (S)-2-[((2R,3R,5R)-4,4-difluoro-3-hydroxy-5-{4-[((S)-1-methylpyrrolidine-3-carbonyl)-amino]-2-oxo-2H-pyrimidin-1-yl}-tetrahydro-furan-2-ylmethoxy)-phenoxy-phosphorylamino)-propionate **(1.1.2(14));**
*iso*-propyl (S)-2-[((2R,3R,5R)-4,4-difluoro-3-hydroxy-5-{4-[((S)-1-methylpyrrolidine-4-carbonyl)-amino]-2-oxo-2H-pyrimidin-1-yl}-tetrahydro-furan-2-ylmethoxy)-phenoxy-phosphorylamino)-propionate **(1.1.2(5));**
*iso*-propyl (S)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(1));**
*iso*-propyl (R)-2-({(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(2));**
*iso*-propyl (R)-2-[((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(3));**
*iso*-propyl (S)-2-((S)-{(2R,3R,4R,5R)-5-[4-(2-dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(4));**
*iso*-propyl (S)-2-((R)-{(2R,3R,4R,5R)-5-[4-(2- dimethylaminoacetylamino)-2-oxo-2H-pyrimidin-1-yl]-4-methyl-4-fluoro-3-hydroxy-tetrahydro-furan-2-ylmethoxy)-phenoxy-phosphorylamino)-propionate **(1.2.2(5));**
*iso*-propyl (S)-2-[(S)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(6));**
*iso*-propyl (S)-2-[(R)-((2R,3R,4R,5R)-3-hydroxy-4-methyl-4-fluoro-5-{4-[((S)-1-methylpyrrolidine-2-carbonyl)-amino]-2-oxo-2H-pyrimidin-1-yl}-tetrahydro-furan-2-ylmethoxy)-phenoxy-phosphorylamino)-propionate **(1.2.2(7)).**

6. A method for preparation of compounds of the general formula **1** and of formula **2,** stereoisomers, pharmaceutically acceptable salts, hydrates, solvates or crystalline forms thereof, consisting in interaction of compound of the general formula **4** with compound of the general formula **5** 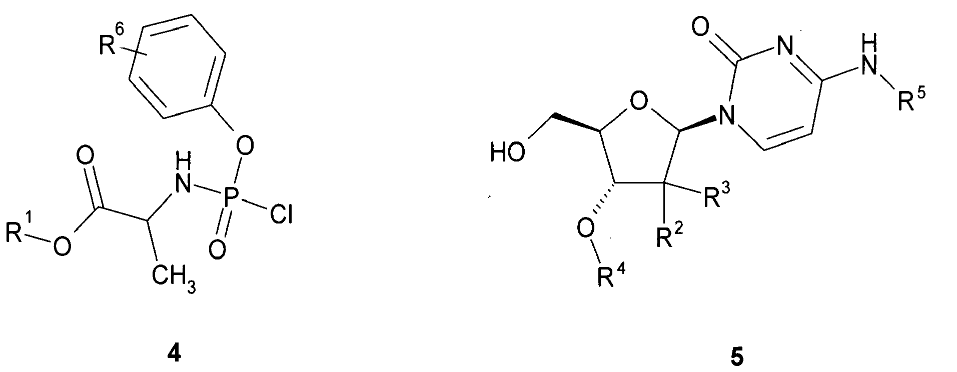 wherein
R¹, R², R³, R⁴, R⁵ and R⁶ have the above meanings.

7. A method for preparation of compounds of the general formula **2,** stereoisomers, pharmaceutically acceptable salts, hydrates, solvates or crystalline forms thereof, consisting in interaction of compound of the general formula **4(1)** with a compound of the general formula **5(1).** 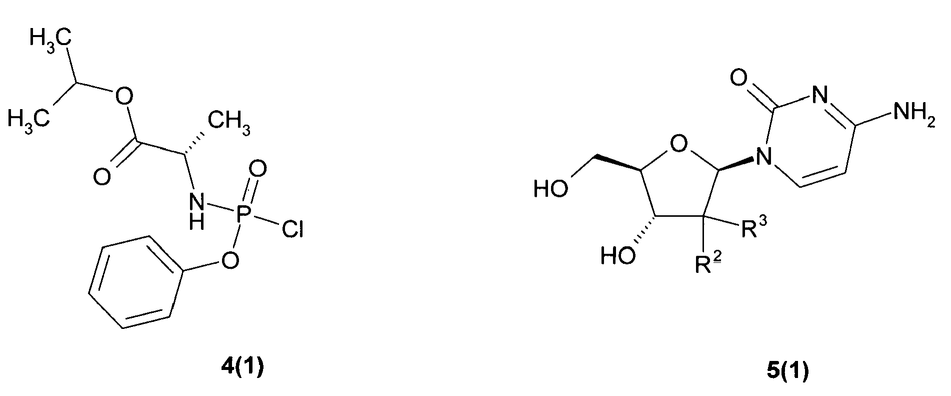 wherein
R², R³ have the above meanings.

8. A method for preparation of compounds of the general formula **1,** stereoisomers, pharmaceutically acceptable salts, hydrates, solvates or crystalline forms thereof, consisting in interaction of compound of the general formula **6** with anhydride of the general formula **7** in presence of 4-dimethylaminopyridine (DMAP) and triethylamine 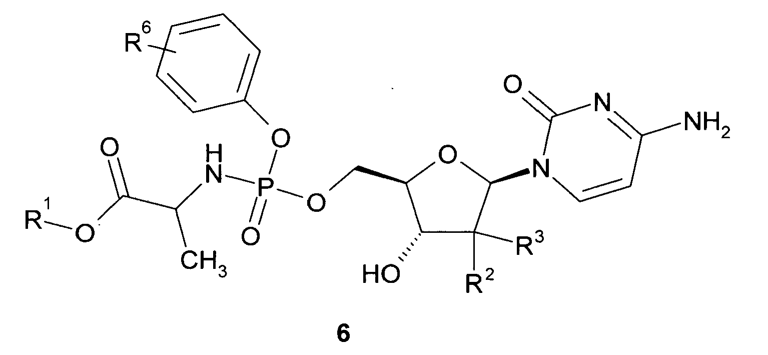
(R⁴)₂O 7
wherein
R⁴ represents optionally substituted α-aminoacyl, and
R⁵ represents hydrogen, and R¹, R², R³ and R⁶ have the above meanings.

9. A method for preparation of compounds of the general formula **3,** stereoisomers, pharmaceutically acceptable salts, hydrates, solvates or crystalline forms thereof, consisting in interaction of compound of the general formula **2** with acetic anhydride in presence of 4-dimethylaminopyridine (DMAP) and triethylamine.

10. A method for preparation of compounds of the general formula **1,** stereoisomers, pharmaceutically acceptable salts, hydrates, solvates or crystalline forms thereof, consisting in interaction of compound of the general formula **6** with α-amino acid of the general formula **8** in presence of 1,1'-carbonyldiimidazole 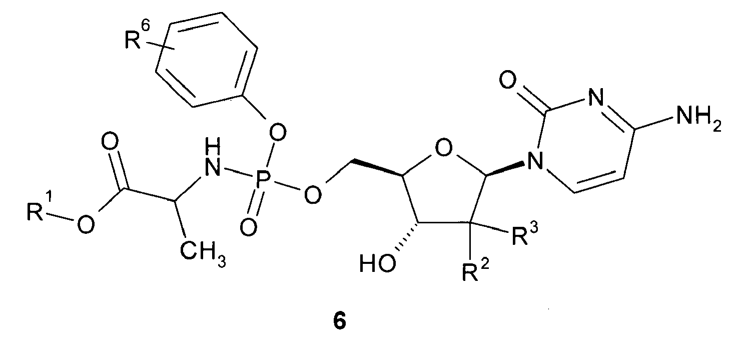
R⁵-OH 8
wherein, R⁴ represents hydrogen, and R⁵ represents optionally substitutede α-aminoacyl, and R¹, R², R³ and R⁶ have the above meanings.

11. A method for preparation of compounds of the general formula **1,** stereoisomers, pharmaceutically acceptable salts, hydrates, solvates or crystalline forms thereof, consisting in interaction of compound of the general formula **9** with an acid of general formula **10** in presence of 1,1'-carbonyldiimidazole and subsequent removal of Boc-protecting group to the resulting product **11** 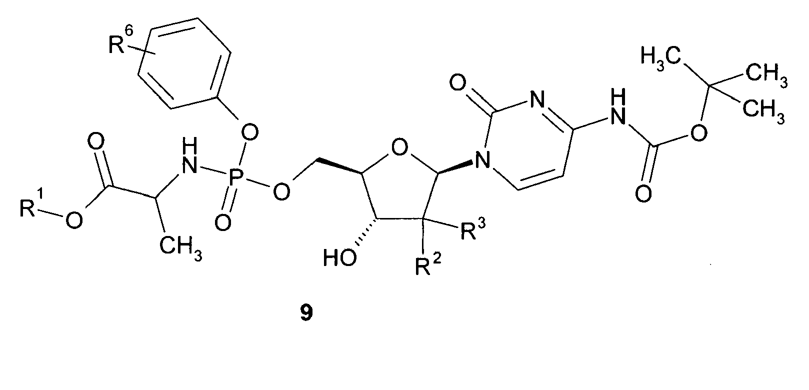
R⁴-OH 10 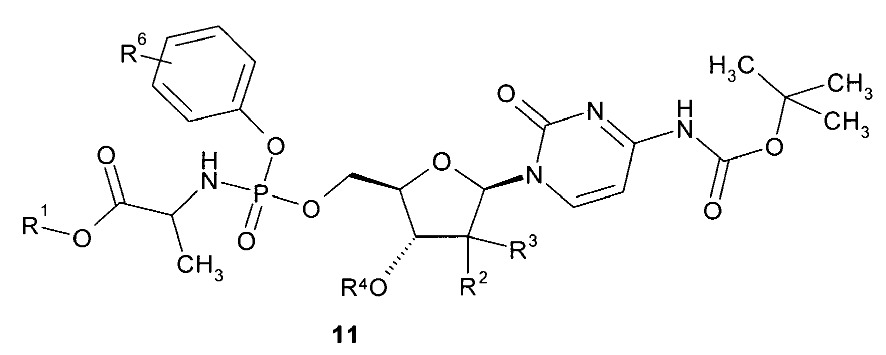
wherein
R⁴ represents optionally substituted α-aminoacyl, and R⁵ represents hydrogen, and
R¹, R², R³ and R⁶ have the above meanings.

12. An active component, exhibiting properties of nucleoside inhibitors of HCV RNA polymerase NS5B, representing compounds of the general formula **1** according to any of claims **1-5,** stereoisomers, pharmaceutically acceptable salts, hydrates, solvates, or crystalline forms thereof.

13. A method for inhibiting of HCV RNA polymerase NS5B by means of compounds of the general formula **1** according to any of claims **1-5,** stereoisomers, pharmaceutically acceptable salts, hydrates, solvates, or crystalline forms thereof.

14. A pharmaceutical composition for prophylaxis and treating viral infections, including hepatisis C comprising in therapeutically effective amount an active component according to claim 12 and optionally comprising an inhibitor of inosine-5-monophosphate dehydrogenase and/or an inhibitor of hepatitis C protease NS3, and/or inhibitor of hepatitis C protease NS3/4A, and/or inhibitor of RNA polymerase NS5A.

15. The pharmaceutical composition according to claim 14 for prophylaxis and treating viral infections, including hepatisis C, in which as an inhibitor of inosine-5-monophosphate dehydrogenase was selected Ribamidin, as an inhibitor of hepatitis C protease NS3 was selected Asunaprevir (BMS-650032), as an inhibitor of hepatitis C protease NS3/4A was selected Sofosbuvir (TMC435), as an inhibitor of RNA polymerase NS5A was selected Daclatasvir (BMS-790052) or Declatasvir (GS-5885).

16. The pharmaceutical composition according to claims 14, 15 for prophylaxis and treating viral infections, including hepatisis C, in the form of tablets, capsuls, injections, ointments, rectal suspensions and gels, placed in in a pharmaceutically acceptable packaging.

17. A medicament for prophylaxis and treating viral infections, including hepatisis C, comprising in effective amount an active component according to claim 12 or pharmaceutical composition according to claims 14-16.

18. A method for for prophylaxis and treatment of disease caused by hepatisis C virus, by introduction of therapeutically effective amount of an active component according to claim 12 or pharmaceutical composition according to claims 14-16 or a medicament according to claim 17.
